# EUROPEAN PATENT APPLICATION

(11) **EP 4 446 332 A1**
(43) Date of publication of application: **16.10.2024**
(21) Application number: 23740309.2
(22) Date of filing: 12.01.2023
(51) Int. Cl.: C07K 1/06, C07F 7/18

(54) **PEPTIDE COMPOUND PRODUCTION METHOD**

(30) Priority: 17.01.2022 JP 2022005195
(71) Applicant: Chubu University Educational Foundation, Kasugai-shi Aichi 487-8501 (JP)
(72) Inventor: YAMAMOTO, Hisashi, Kasugai-shi, Aichi 487-8501 (JP); HATTORI, Tomohiro, Kasugai-shi, Aichi 487-8501 (JP)
(74) Representative: Lavoix
(86) International application number: PCT/JP2023/000653
(87) International publication number: WO 2023/136301

(57) **Abstract**

Provided is a method that prevents decreases in solubility in an organic solvent when elongating a peptide chain by a peptide coupling reaction and improves the reactivity of a peptide chain and the operability in a purification process. The method makes it possible to obtain a polypeptide compound represented by formula (P1) by performing amide formation reaction of the carboxyl group on the right side of the amino acid or the peptide compound in formula (R1) with the amino group on the left side of the amino acid or the peptide ester compound in formula (R2) in which the terminal carboxyl group is protected by a silicon-containing hydrophobic substituent TAG(Si).

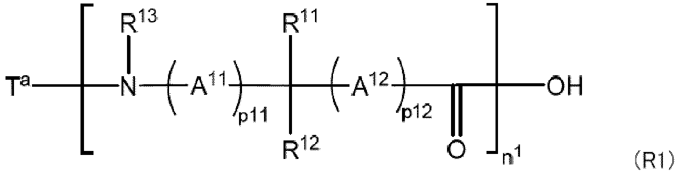

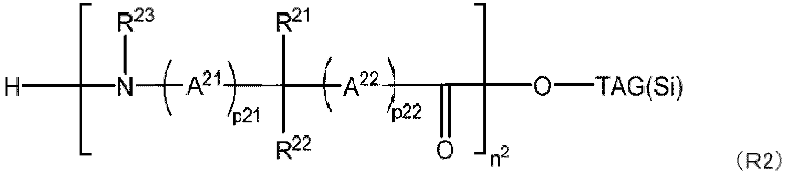

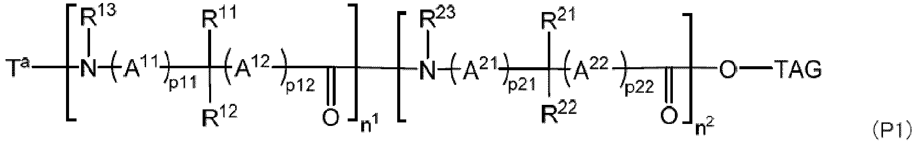

## Description

### FIELD

The present invention relates to a novel method for producing a peptide compound.

### BACKGROUND

Conventionally, amide compounds represented by peptides have been used in a wide variety of fields, including pharmaceuticals, cosmetics, and functional foods. Development of synthetic methods thereof has been diligently pursued as an important research goal in synthetic chemistry (Non-Patent Literature 1 to 3). However, there are few truly effective catalysts or reaction agents other than carboxylic acid activators for the amidation reaction, which is the most important reaction in peptide synthesis. Therefore, it is unavoidable to use a reaction mode that forms by-products, and thus, peptide synthesis, which involves repeating multi-stage reactions, is extremely inefficient from the viewpoint of atom economy (atomic yield). The amount of by-products is large, and there are few effective purification means. As a result, the cost of disposal of by-products and purification constitutes most of the necessary costs for peptide synthesis, and is the largest obstacle to development in this field.

In peptide synthesis, which uses amino acids or derivatives thereof as starting materials, it is desirable for the amidation reaction to proceed with high stereoselectivity. Enzyme reactions in the body are examples of highly stereoselective amidation reactions. For example, in the body, peptides are synthesized with extremely high stereoselectivity through sophisticated use of enzymes and hydrogen bonds. However, enzyme reactions are not suitable for mass production, requiring enormous financial and time costs when applied to synthetic chemistry.

In synthetic chemistry, amidation reactions using catalysts have been examined, but in conventional means, the amide bond is formed primarily through the method of activating carboxylic acid, such that racemization occurs quickly, whereby synthesizing a peptide with high stereoselectivity and efficiency is difficult.

According to conventional methods, it is very difficult to link an additional amino acid or derivative to a peptide comprising a plurality of amino acids or derivatives thereof (chemical ligation) or link two or more peptides via amide bonds. As an amidation method for ligation to such peptides, there are known a method for ligation by using an amino acid having a sulfur atom to utilize the high reactivity of the sulfur atom (Non-Patent Literature 4) and a method for ligation by synthesizing an amino acid hydroxyamine to utilize the high reactivity of the hydroxyamine (Non-Patent Literature 5). However, in the former method, it is difficult to synthesize amino acids having a sulfur atom, and in the latter method, hydroxyamine synthesis involving several steps is necessary. Both methods are time-consuming and costly and have a disadvantage in efficiency.

The present inventors have developed, as techniques for synthesizing an amide compound in a highly chemoselective manner: a method of amidating a carboxylic acid/ester compound having a hydroxy group at the β-position in the presence of a specific metal catalyst (Patent Literature 1); a method of using a hydroxyamino/imino compound as an amino acid precursor and amidating it in the presence of a specific metal catalyst, and then reducing them in the presence of a specific metal catalyst (Patent Literature 2); and a method of amidating a carboxylic acid/ester compound in the presence of specific metal catalyst (Patent Literature 3). The present inventors have also developed a technique for highly efficient and selective synthesis of peptides consisting of various amino acid residues by amide reaction of the carboxyl group of an N-terminal protected amino acid/peptide and the amino group of a C-terminal protected amino acid/peptide in the presence of a specific silylating agent and an optionally used Lewis acid catalyst, followed by deprotection (Patent Literature 4). The present inventors have further developed a method of synthesizing a peptide composed of various amino acid residues with a high efficiency and in a highly selective manner, by causing an amide reaction a carboxyl group of an amino acid or peptide whose N-terminal is either protected or unprotected and an amino group of an amino acid or peptide whose C-terminal is either protected or unprotected in the presence of a specific silylating agent, followed by deprotection (Patent Literatures 5 and 6), and a method for causing an amidation reaction using a Bronsted acid as a catalyst (Patent Literature 7).

Most of the peptide drugs currently on the market are oligopeptides with five or more amino acid residues. As a peptide chain is elongated by the peptide bond reaction, the polarity of the peptide becomes significantly higher, and its solubility in an organic solvent used as the reaction solvent is significantly reduced. This significantly reduces the reactivity of the peptide chain and is also a major disadvantage in terms of operability during the purification process. Therefore, a strategy that ensures solubility in organic solvents during the peptide chain elongation reaction is essential for the synthesis of oligopeptides.

A known technique to ensure the solubility and reactivity of a peptide chain in an organic solvent includes linking an alkoxybenzyl ester (TAGa) group, which is a long-chain alkyl linked to an aromatic ring, to the peptide chain as a protective group (Non-Patent Literature 6). However, this technique had limited effect in improving the solubility of peptide chains in organic solvents. In addition, the present inventors have observed that this technique causes insoluble solids in the reaction process, suggesting that deprotection of the ester may occur in the reaction process.

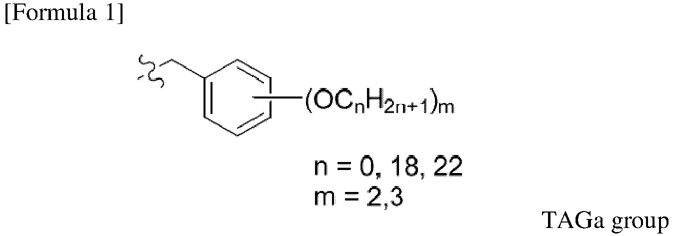

Another known technique includes linking a silyl ester group to the peptide chain as a protecting group (Non-Patent Document 7). However, this technique uses a highly reactive silyl ester group as a protecting group, which significantly limits the reaction conditions applicable to peptide bonding reactions. In addition, there is a possibility that deprotection of the ester may occur in the reaction process in this technique as well, and in fact, the occurrence of deprotection was frequently confirmed and reported in this literature.

### CITATION LIST

### Patent Literature

[Patent Literature 1] WO2017/204144 A
[Patent Literature 2] WO2018/199146 A
[Patent Literature 3] WO2018/199147 A
[Patent Literature 4] WO2019/208731 A
[Patent Literature 5] WO2021/085635 A
[Patent Literature 6] WO2021/085636 A
[Patent Literature 7] WO2021/149814 A

### Non-Patent Literature

[Non-Patent Literature 1] Chem. Rev., 2011, Vol.111, p.6557-6602
[Non-Patent Literature 2] Org. Process Res. Dev., 2016, Vol.20, No.2, p.140-177
[Non-Patent Literature 3] Chem. Rev., 2016, Vol.116, p.12029-12122
[Non-Patent Literature 4] Science, 1992, Vol.256, p.221-225
[Non-Patent Literature 5] Angew. Chem. Int. Ed., 2006, Vol.45, p.1248-1252
[Non-Patent Literature 6] Org. Process Res. Dev. 2019, Vol.23, p.2576-2581
[Non-Patent Literature 7] Org. Process Res. Dev. 2021, Vol.25, p.2029-2038

### SUMMARY

### Problem to be Solved by the Invention

### SUMMARY

### Problem to be Solved by the Invention

Against this background, there is a need for a method that can improve the reactivity of peptide chains and the operability of the purification process by preventing a decrease in solubility in organic solvents during the elongation of peptide chains by the peptide compound binding reaction. The present invention has been made in view of these issues.

### Means for Solving the Problem

As a result of intensive investigations, the present inventors have found that by using amino acids or peptides whose terminal carboxyl and/or amino groups are protected by silicon-containing hydrophobic substituents having specific structures for the elongation of peptide chains by peptide bonding reactions, it is possible to prevent a decrease in solubility of the peptide chains in organic solvents and to maintain and improve the solubility of the peptide chains in the organic solvents, whereby the present inventors have arrived at the present invention.

Thus, the present invention provides the following aspects.

### [Aspect 1]

A method for producing a polypeptide compound, comprising the step of causing an amide forming reaction between the carboxyl group on the right side of an amino acid or peptide compound represented by formula (R1) and the amino group on the left side of represented by formula (R2) amino acid ester or peptide ester compound to produce a peptide compound represented by formula (P1). 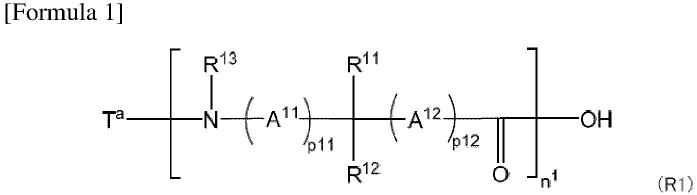 In formula (R1),
T^{a} represents a hydrogen atom or monovalent substituent,
R¹¹ and R¹² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
R¹³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R¹¹ and R¹³ may be bound to each other to form, together with the carbon atom to which R¹¹ binds and the nitrogen atom to which R¹³ binds, a heterocyclic group that may have one or more substituents,
A¹¹ and A¹² each represent, independently of each other, a divalent aliphatic hydrocarbon group that may have one or more substituents having 1 to 3 carbon atoms,
p11 and p12 each represent, independently of each other, 0 or 1, and
n¹ represents an integer of equal to or greater than 1 corresponding to the number of the structure units parenthesized with [ ], provided that when n¹ is equal to or greater than 2, then the two or more structure units in [ ] may be either identical to each other or different from each other. 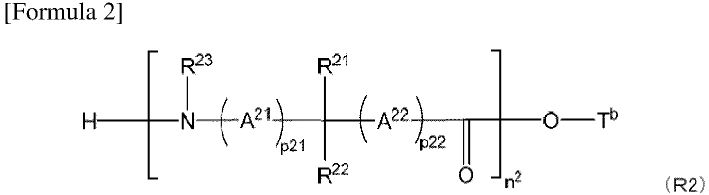
In formula (R2),
   T^{b} represents a hydrogen atom or monovalent substituent,
   R²¹and R²² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
   R²³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
   R²¹ and R²³ may be bound to each other to form, together with the carbon atom to which R²¹ binds and the nitrogen atom to which R²³ binds, a heterocyclic group that may have one or more substituents,
   A²¹ and A²² each represent, independently of each other, a divalent aliphatic hydrocarbon group that may have one or more substituents having 1 to 3 carbon atoms,
   p21 and p22 each represent, independently of each other, 0 or 1, and
   n² represents an integer of equal to or greater than 1 corresponding to the number of the structure units parenthesized with [ ], provided that when n² is equal to or greater than 2, then the two or more structure units in [ ] may be either identical to each other or different from each other, provided that
      (i) T^{a} is a group represented by -O-C(=O)-TAG(Si) and/or
      (ii) T^{b} is a group represented by -TAG(Si),
   where -TAG(Si) has the following structure. 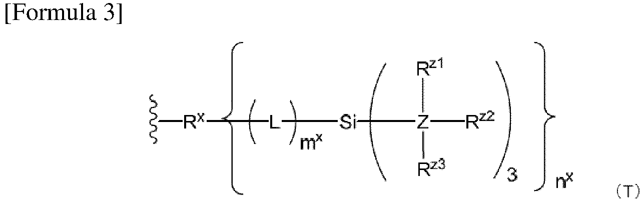
   In formula (T),
      R^{x} represents a divalent, trivalent, or tetravalent aliphatic hydrocarbon group, aromatic hydrocarbon group, or heterocyclic group that may have one or more substituents,
      L represents a single bond or a divalent linking group,
      m^{x} represents an integer of 0 or 1 provided that when m^{x} is 0, L is a single bond,
      Z represent, independently of each other, a carbon atom (C) or a silicon atom (Si),
      R^{z1}, R^{z2}, and R^{z3} represent, independently of each other, a monovalent aliphatic hydrocarbon group, aromatic hydrocarbon group, silicon-containing hydrocarbon group, or heterocyclic group that may have one or more substituents,
      provided that the three parenthesized substituents in -Z(R^{z1})(R^{z2})(R^{z3}) may either be the same as each other or different from each other,
      n^{x} represents an integer of 1 to 3 corresponding to the number of the substituents
      parenthesized with { }, provided that when m^{x} is 2 or 3, then the two or three substituents parenthesized with { } in formula (T) may either be the same as each other or different from each other. 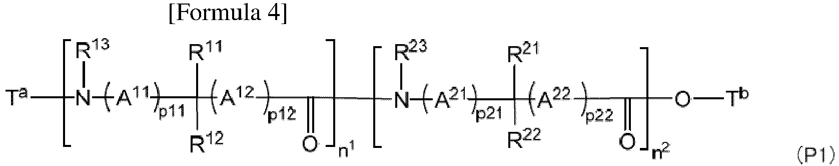
      In formula (P1),
         T^{a}, R¹¹, R¹², R¹³, A¹¹, A¹², p11, p12, and n¹ each represent the same definitions as those of the same symbols in general formula (R1) above, and
         R²¹, R²², R²³, A²¹, A²², p21, p22, n², and T^{b} each represent the same definitions as those of the same symbols in general formula (R2) above.

### [Aspect 2]

The method according to Aspect 1, wherein -TAG(Si) has a structure represented by formula (T1), (T2), or (T3) below. 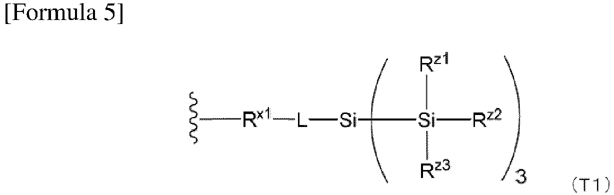 In formula (T1),
R^{x1} represents a divalent aliphatic hydrocarbon group, aromatic hydrocarbon group, or heterocyclic group that may have one or more substituents, and
L, R^{z1}, R^{z2}, and R^{z3} represent, independently of each other, the same definitions as in formula (T). 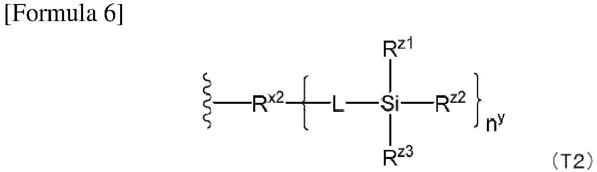
In formula (T2),
   R^{x2} represents a trivalent or tetravalent aliphatic hydrocarbon group, aromatic hydrocarbon group, or heterocyclic group that may have one or more substituents,
   L, R^{z1}, R^{z2}, and R^{z3} represent, independently of each other, the same definitions as in formula (T), and
   n^{y} represents an integer of 2 or 3 corresponding to the number of the substituents parenthesized with { }, provided that the two or three substituents parenthesized with { } in formula (T2) may either be the same as each other or different from each other . 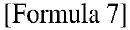 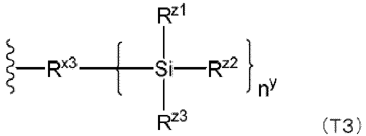
   In formula (T3),
      R^{x3} represents a trivalent or tetravalent aliphatic hydrocarbon group, aromatic hydrocarbon group, or heterocyclic group that may have one or more substituents,
      R^{z1}, R^{z2}, and R^{z3} represent, independently of each other, the same definitions as in formula (T), and
      n^{y} represents an integer of 2 or 3 corresponding to the number of the substituents parenthesized with { }, provided that the two or three substituents parenthesized with { } in formula (T3) may either be the same as each other or different from each other.

### [Aspect 3]

The method according to Aspect 1 or 2, wherein the reaction is carried out in an organic solvent.

### [Aspect 4]

The method according to any one of Aspects 1 to 3, wherein the reaction is carried out in the presence of a Lewis acid catalyst.

### [Aspect 5]

The method according to Aspect 4, wherein the Lewis acid catalyst is a metal compound containing one or more metals selected from the group consisting of aluminum, titanium, zirconium, hafnium, tantalum, and niobium.

### [Aspect 6]

The method according to any one of Aspects 1 to 5, wherein the reaction is carried out in the presence of a phosphorus compound.

### [Aspect 7]

The method according to Aspect 6, wherein the phosphorus compound is a phosphine compound or a phosphate compound.

### [Aspect 8]

The method according to any one of Aspects 1 to 7, wherein the reaction is carried out in the presence of a silane compound.

### [Aspect 9]

The method according to any one of Aspects 1 to 8, wherein the T^{a} group in formula (R1) is -O-C(=O)-TAG(Si), and the method further comprises the step of, after the synthesis of the peptide compound of formula (P1), deprotecting the terminal amino group of the peptide compound by removing the -O-C(=O)-TAG(Si) group from the peptide compound.

### [Aspect 10]

The method according to any one of Aspects 1 to 9, wherein the T^{b} group in formula (R1) is -TAG(Si), and the method further comprises the step of, after the synthesis of the peptide compound of formula (P1), deprotecting the terminal carboxyl group of the peptide compound by removing the -TAG(Si) group from the peptide compound.

### [Aspect 11]

The method according to any one of Aspects 1 to 10, wherein the reaction is carried out as a batch reaction or a flow reaction.

### [Aspect 12]

An amino acid or peptide compound represented by formula (R1') below. 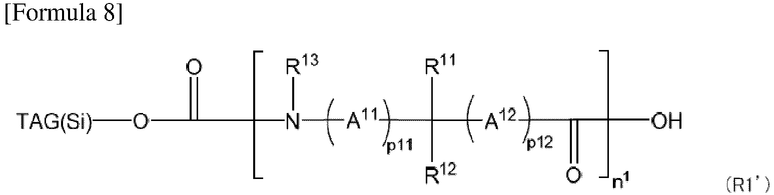 In formula (Rl'), R¹¹, R¹², R¹³, A¹¹, A¹², p11, p12, n¹, and TAG(Si) each represent the same definitions as those of the same symbols in Aspect 1.

### [Aspect 13]

An amino acid ester or peptide ester compound represented by formula (R2') below. 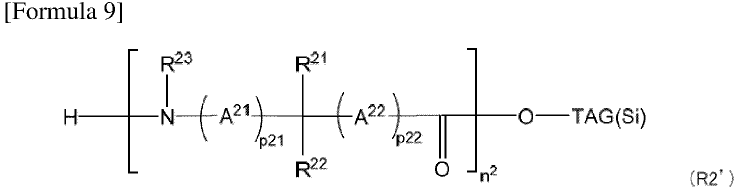 In formula (R2'), R²¹, R²², R²³, A²¹, A²², p21, p22, n², and TAG(Si) each represent the same definitions as those of the same symbols in Aspect 1.

### [Aspect 14]

A peptide compound represented by formula (P1') below. 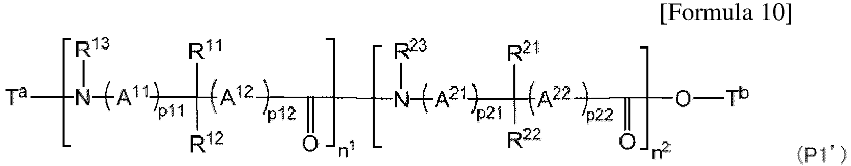 In formula (P1'),
R¹¹, R¹², R¹³, R²¹, R²², R²³, A¹¹, A¹², A²¹, A²², p21, p22, p11, p12, n¹, n², T^{a}, and T^{b} each represent the same definitions as those of the same symbols in Aspect 1, provided that
   (i) T^{a} is a group represented by -O-C(=O)-TAG(Si), and/or,
   (ii) T^{b} is a group represented by -TAG(Si),
where -TAG(Si) represents the same definition as in Aspect 1.

### [Aspect 15]

A compound represented by formula (C0) below.
[Formula 11]

TAG(Si)-OH (C0)

In formula (C0), -TAG(Si) represents the same definition as the same symbol in Aspect 1.

### [Aspect 16]

A compound represented by formula (C1) below. 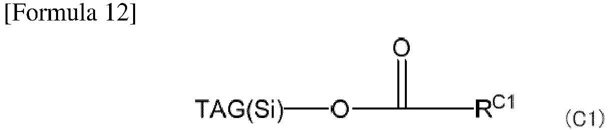 In formula (C1),
-TAG(Si) represents the same definition as the same symbol in Aspect 1, and
R^{C1} represents a monovalent group.

### [Aspect 17]

A compound represented by formula (C2) below. 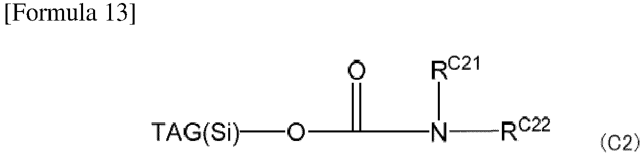 In formula (C2),
- TAG(Si) represents the same definition as the same symbol in Aspect 1, and R^{C21} and R^{C22} represent, independently of each other, a monovalent group.

### Effects of the Invention

According to the method of the present invention, amino acids or peptides whose terminal carboxyl group and/or terminal amino group are protected by a silicon-containing hydrophobic substituents having a specific structure are subjected to peptide chain elongation by peptide bond reaction. This can prevent the peptide chain from losing its solubility in organic solvents, thereby improving the reactivity of the peptide chain and the ease of use in the purification process.

### BRIEF EXPLANATION OF THE DRAWINGS

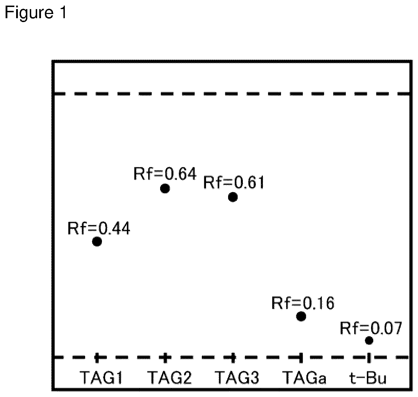

[Figure 1] Figure 1 is a graph showing the results of evaluation in which silicon-containing hydrophobic substituents TAG 1 (Si), TAG2(Si), and TAG3(Si), which were synthesized in Examples 1 to 3, tBu group, which is a known protecting group, and TAGa group, which is a protecting group described in Non-Patent Literature 6 (Org. Process Res. Dev. 2019, Vol.23, p.2576-2581), were used to protect the terminal carboxyl group of an amino acid (L-Ala) and the resulting decrease in the polarity was evaluated.

### EMBODIMENTS

The present invention is described hereinafter in detail with reference to specific embodiments thereof. However, the present invention is not limited to the following embodiments and can be carried out in any embodiment that does not deviate from the gist according to the present invention.

All patent publications, patent application publications, and non-patent documents cited in this disclosure are incorporated herein by reference in their entirety for all purposes.

### [I. Definitions of Terms]

The term "amino acid" herein refers to a compound having a carboxyl group and an amino group. Unless otherwise specified, the type of an amino acid is not particularly limited. For example, from the viewpoint of optical isomerism, an amino acid may be in the D-form, in the L-form, or in a racemic form. From the viewpoint of the relative positions of the carboxyl group and the amino group, an amino acid may be any of an α-amino acid, β-amino acid, γ-amino acid, δ-amino acid, or ε-amino acid. Examples of amino acids include, but are not limited to, natural amino acids that make up proteins. Examples include valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, and serine.

The term "peptide" herein refers to a compound comprising a plurality of amino acids linked together via peptide bonds. Unless otherwise specified, the plurality of amino acid units constituting a peptide may be the same type of amino acid unit or may consist of two or more types of amino acid units. The number of amino acids constituting a peptide is not restricted as long as it is two or more. Examples include 2 (also called "dipeptide"), 3 (also called "tripeptide"), 4 (also called "tetrapeptide"), 5 (also called "pentapeptide"), 6, 7, 8, 9, 10, 15, 20, 30, 40, 50, 100, or more. The term "polypeptide" may also be used to refer to tripeptides and longer peptides.

The term "amino group" herein refers to a functional group represented by any formula of -NH₂, -NRH, and -NRR' (where R and R' each represent a substituent) obtained by removing hydrogen from ammonia, a primary amine, and a secondary amine, respectively.

Unless otherwise specified, a hydrocarbon group herein may be either aliphatic or aromatic. An aliphatic hydrocarbon group may be in the form of either a chain or a ring. A chain hydrocarbon group may be linear or branched. A cyclic hydrocarbon group may be monocyclic, bridged cyclic, or spirocyclic. The hydrocarbon group may be saturated or unsaturated. In other words, one, two, or more carbon-carbon double and/or triple bonds may be included. Specifically, "hydrocarbon group" represents a concept including an alkyl group, alkenyl group, alkynyl group, cycloalkyl group, cycloalkenyl group, cycloalkynyl group, aryl group, arylalkyl group, alkylaryl group, etc. Unless otherwise specified, one, two, or more hydrogen atoms of the hydrocarbon group may be replaced with any substituents and one, two, or more carbon atoms in the hydrocarbon group may be replaced with any heteroatoms corresponding to the valence thereof.

The term "hydrocarbon oxy group" herein refers to a group comprising an oxy group (-O-) linked via one bond thereof to the hydrocarbon group as defined above.

The term "hydrocarbon carbonyl group" herein refers to a group comprising a carbonyl group (-C(=O)-) linked via one bond thereof to the hydrocarbon group as defined above.

The term "hydrocarbon sulfonyl group" herein refers to a group comprising a sulfonyl group (-S(=O)₂-) linked via one bond thereof to the hydrocarbon group as defined above.

A heterocyclic group may be saturated or unsaturated. In other words, it may contain one, two, or more carbon-carbon double and/or triple bonds. A heterocyclic group may be monocyclic, bridged cyclic, or spirocyclic. The heteroatom included in the constituent atoms of the heterocyclic group is not particularly limited, examples thereof including nitrogen, oxygen, sulfur, phosphorus, and silicon.

The term "heterocyclic oxy group" herein refers to a group comprising an oxy group (-O-) linked via one bond thereof to the heterocyclic group as defined above.

The term "heterocyclic carbonyl group" herein refers to a group comprising a carbonyl group (-C(=O)-) linked via one bond thereof to the heterocyclic group as defined above.

The term "heterocyclic sulfonyl group" herein refers to a group comprising a sulfonyl group (-S(=O)₂-) linked via one bond thereof to the heterocyclic group as defined above.

The term "metalloxy group" (that may have one or more substituents) herein refers to a group represented by formula (R)ₙ-M-O-, where M refers to a metal element, R refers to a substituent, n refers to an integer of 0 or more but 8 or less corresponding to the coordination number of the metal element M.

Unless otherwise specified, the term "substituent" herein refers, independently of each other, to any substituent which is not particularly limited so long as the amidation step of the production method according to the present invention proceeds. Examples include, but are not limited to, a halogen atom, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyloxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyloxy group, heterocyclic oxy group, heterocyclic carbonyl group, heterocyclic oxycarbonyl group, heterocyclic carbonyloxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyloxy group. The term "substituents" also may include functional groups obtained by substituting any of the aforementioned functional groups with any of the aforementioned functional groups as long as the valence and physical properties thereof permit. When a functional group has one or more substituents, the number of the substituents is not particularly limited as long as the valence and physical properties thereof permit. When the functional group has two or more substituents, they may be identical to each other or different from each other.

The main abbreviations used herein are listed in Table 1 below.

**[Table 1]**

| Abbreviation | Definition |
|---|---|
| Ac | acetyl |
| acac | acetyl acetonate |
| Alloc | allyoxycarbonyl |
| Bn or Bzl | benzyl |
| Boc | tert-butoxycarbonyl |
| Bu | butyl |
| Bz | benzoyl |
| Cbz | benzyloxycarbonyl |
| Cp | cyclopentadienyl |
| 2,4-DNP | 2,4-dinitrophenyl |
| Et | ethyl |
| Fmoc | 9-fluorenylmethyloxycarbonyl |
| HOBt | 1-hydroxybenzotriazole |
| i-Pr | isopropyl |
| MABR | methylaluminum bis(4-bromo-2,6-di-tert-butylphenoxyde) |
| Me | methyl |
| Ms | mesyl |
| Ns | 2 or 4-nitrobenzenesulfonyl |
| Phth | phthaloyl |
| PMB | paramethoxybenzyl |
| PMPCO | paramethoxybenzoyl |
| Pr | propyl |
| TBAF | tetrabutylammonium fluoride |
| TBDPS | tert-butyl diphenylsilyl |
| TBS | tritert-butylsilyl |
| t-Bu | tert-butyl |
| TES | triethylsilyl |
| Tf | trifluoromethane sulfonyl |
| THF | tetrahydrofuran |
| TIPS | triisopropylsilyl |
| TMS | trimethylsilyl |
| TMS-IM | trimethylsilyl imidazole |
| TMS-OTf | trimethylsilyl trifluoromethanesulfonate |
| Troc | 2,2,2-trichloroethoxy carbonyl |
| Trt | trityl |
| Ts | toluenesulfonyl |
| wscHCl | 1-ethyl-3-(3-dimethylaminopropyl)carbodiimide hydrochloride |

Amino acids and residues thereof may herein be represented by three-letter abbreviations well known to a person skilled in the art. The three-letter abbreviations of major amino acids are shown in the following table.

**[Table 2]**

| Abbreviation | Definition |
|---|---|
| Ala | Alanine |
| Arg | Arginine |
| Asn | Asparagine |
| Asp | Aspartic acid |
| Cys | Cysteine |
| Gln | Glutamine |
| Glu | Glutamic acid |
| Gly | Glycine |
| His | Histidine |
| Ile | Isoleucine |
| Leu | Leucine |
| Lys | Lysine |
| Met | Methionine |
| Phe | Phenylalanine |
| Phg | Phenylglycine |
| Pro | Proline |
| Ser | Serine |
| Thr | Threonine |
| Trp | Tryptophan |
| Tyr | Tyrosine |
| Val | Valine |

β-homoamino acids and residues thereof may herein be represented by "Ho" followed by three-letter abbreviations of corresponding β-amino acids.

### [II. Production Method of Peptide Compounds]

### * Summary

An embodiment according to the present invention relates to a method for producing a polypeptide compound, the method including causing an amide forming reaction between the carboxyl group on the right side of an amino acid or peptide compound represented by formula (R1) and the amino group on the left side of represented by formula (R2) amino acid ester or peptide ester compound to produce a peptide compound represented by formula (P1) (hereinafter also referred to as "the production method (1) of peptide compounds according to the present invention" or simply as "the production method (1) of the present invention").

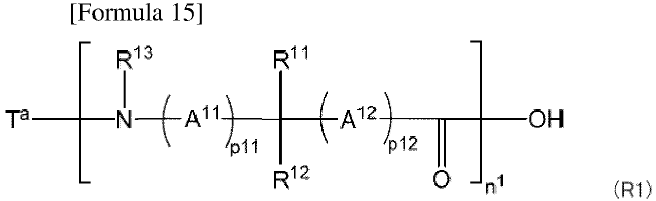

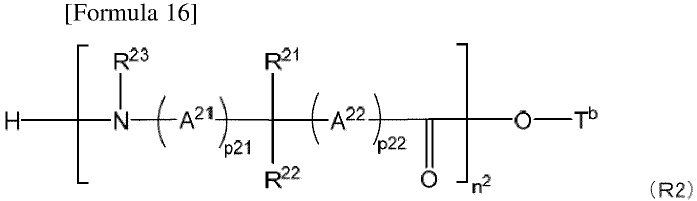

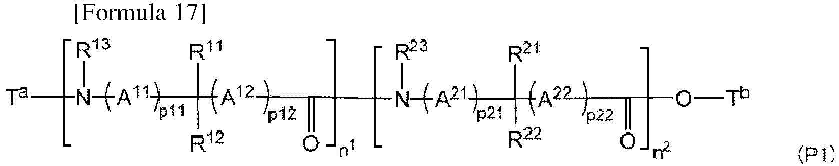

### *Substrate Compounds

The symbols in formulae (R1) and (R2) are defined as follows.

R¹¹, R¹², R²¹, and R²² each represent, independently of each other, a hydrogen atom, halogen atom, hydroxyl group, carboxyl group, nitro group, cyano group, or thiol group, or a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

When each of R¹¹, R¹², R²¹, and/or R²² is a monovalent hydrocarbon group or heterocyclic group that may have one or more substituents, a linking group may intervene between the hydrocarbon group or heterocyclic group and the carbon atom to which it binds. The linking group may be, independently of each other, selected from, although is not limited to, the structures listed below (where, in the chemical formulae below, A represents, independently of each other, a monovalent hydrocarbon group or heterocyclic group that may have one or more substituents. When two A's are present in the same group, they may be identical to each other or different from each other.).

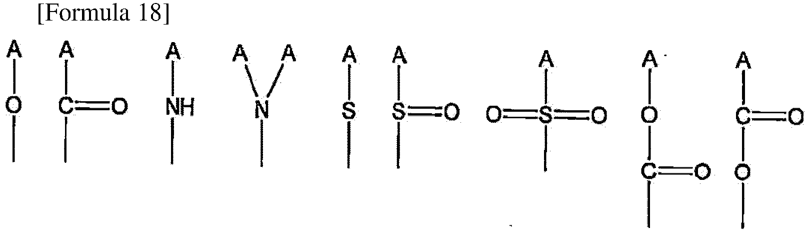

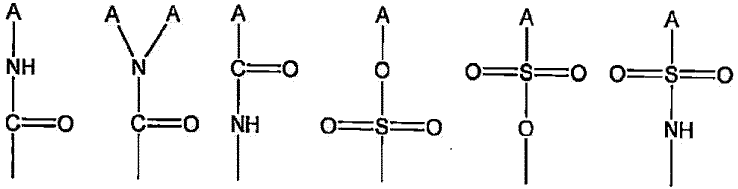

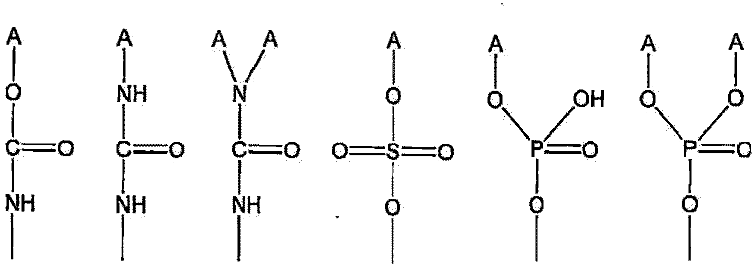

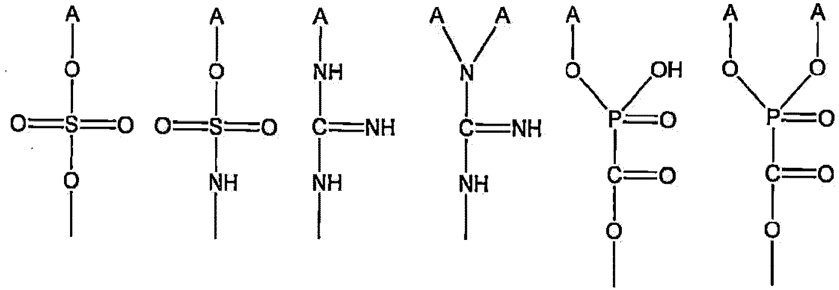

The number of carbon atoms in the hydrocarbon group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The total number of carbon atoms and hetero atoms in the heterocyclic group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the heterocyclic structure, but may be 3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Among them, each of R¹¹, R¹², R²¹, and R²² may preferably be, independently of each other, a hydrogen atom, hydroxyl group, thiol group, carboxyl group, nitro group, cyano group, or halogen atom, or an amino group, alkyl group, alkenyl group, cycloalkyl group, alkoxy group, aryl group, aryloxy group, acyl group, heterocyclic group, or heterocyclic oxy group that may have one or more substituents.

Specific examples of R¹¹, R¹², R²¹, and R²² may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethylamino group, benzylamino group, and triphenylmethylamino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyl oxy group, pyrrolyl oxy group, indolyl oxy group, and quinolyl oxy group.

Of the groups mentioned above, those having a carboxyl group may or may not have a protective group. Although it depends on the reactivity of the compound of formula (R1) and the compound of formula (R2) used in the reaction, if the carboxyl group in any of the above substituents has a protective group, the reaction selectivity with the carboxyl ester group on the right side of the compound represented by formula (R2) may usually be improved over that with the carboxyl group present on the other substituents.

R¹³and R²³ each represent, independently of each other, a hydrogen atom, carboxyl group, or hydroxyl group, or a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

When each of R¹³and/or R²³ is a monovalent hydrocarbon group or heterocyclic group that may have one or more substituents, a linking group may intervene between the hydrocarbon group or heterocyclic group and the nitrogen atom to which it binds. The linking group may be, independently of each other, selected from, although is not limited to, the structures listed below (where, in the chemical formulae below, A represents, independently of each other, a monovalent hydrocarbon group or heterocyclic group that may have one or more substituents. When two A's are present in the same group, they may be identical to each other or different from each other.).

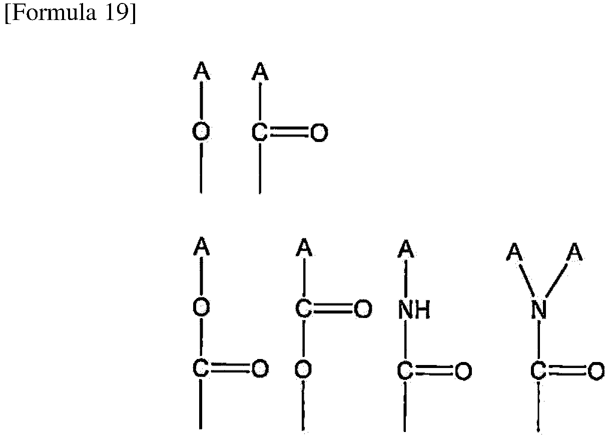

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Each of R¹³and R²³ may preferably be, independently of each other, a hydrogen atom, hydroxyl group, or carboxyl group, or an alkyl group, alkenyl group, cycloalkyl group, alkoxy group, aryl group, aryloxy group, acyl group, heterocyclic group, or heterocyclic oxy group that may have one or more substituents.

Specific examples of R¹³and R²³ may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethyl amino group, benzyl amino group, and triphenylmethyl amino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyl oxy group, pyrrolyl oxy group, indolyl oxy group, and quinolyl oxy group.

Alternatively, R¹¹ and R¹³ may be bound to each other to form, together with the carbon atom to which R¹¹ binds and the nitrogen atom to which R¹³ binds, a hetero ring that may have one or more substituents, and R²¹ and R²³ may be bound to each other to form, together with the carbon atom to which R²¹ binds and the nitrogen atom to which R²³ binds, a hetero ring that may have one or more substituents. If these groups have one or more substituents, they may be selected arbitrarily from those detailed earlier. The number of substituents may be 5, 4, 3, 2, 1, or 0.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

A¹¹, A¹², A²¹, and A²² each represent, independently of each other, a divalent aliphatic hydrocarbon group containing 1 to 3 carbon atoms that may have one or more substituents. Specific Examples include, although are not limited to, methylene group, ethylene group, propylene group, and isopropylene group, as well as groups derived from these groups via substitution with one or more substituents mentioned above. Specific examples of the number of substituents are 3, 2, 1, or 0.

p11, p12, p21, and p22 each represent, independently of each other, 0 or 1.

n¹ represents the number of amino acid units parenthesized with [ ] in formula (R1), and is an integer of 1 or more. When n¹ is 1, the compound of formula (R1) is an amino acid, and when n¹ is 2 or more, the compound of formula (R1) is a peptide. The upper limit for n¹ is not particularly limited so long as the amidation step proceeds, but may preferably be, for example, 100 or less, 80 or less, 60 or less, 50 or less, 40 or less, 30 or less, 20 or less, 15 or less, 12 or less, or 10 or less. Specific examples of n¹ may be 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100.

n² represents the number of amino acid units parenthesized with [ ] in formula (R2), and is an integer of 1 or more. When n² is 1, the compound of formula (R2) is an amino acid, and when n² is 2 or more, the compound of formula (R2) is a peptide. The upper limit for n² is not particularly limited so long as the amidation step proceeds, but may preferably be, for example, 1, 2, 3, 4, 5, 6, 7, 8, 9, or 10. However, n² may preferably be 1, i.e., the compound of formula (R2) may preferably be an amino acid. Although not bound by any theory, the present inventors speculate that when n² is 1, that is, when the compound of formula (R2) is an amino acid, it may react with the aluminum compound of formula (A) to form a cyclization intermediate containing an aluminum atom, which is one factor that improves the reaction efficiency.

Needless to say, when n¹ is equal to or greater than 2, then R¹¹, R¹², R¹³, A¹¹, A¹², p11, and p12, which define the structure units parenthesized with [ ], may be either identical to each other or different from each other between the two or more amino acid units. Likewise, when n² is equal to or greater than 2, then R²¹, R²², R²³, A²¹, A²², p21, and p22, which define the structure units parenthesized with [ ], may be either identical to each other or different from each other between the two or more amino acid units. In other words, when each of the compound of formula (R1) and/or the compound of formula (R2) is a peptide, then the two or more amino acid units constituting the peptide may be either identical to each other or different from each other.

T^{a1} in Compound (R1) represents a hydrogen atom or monovalent substituent. When it is a monovalent substituent, specific examples may include, although are not limited to, the groups mentioned as examples for R¹³ and R²³ above, as well as protective groups for amino groups (hereinafter also referred to as PG^{a}), and the silicon-containing hydrophobic substituent -TAG(Si), which will be explained later. The protective group for amino groups PG^{a} is not restricted as long as the amino group can be protected so that it does not react during the amidation reaction and can be deprotected and converted to an amino group after the reaction. The details of the protective groups for amino groups PG^{a} and the silicon-containing hydrophobic substituent -TAG(Si) will be explained later.

PG^{b} in Compound (R2) represents a protective group for carboxyl groups. When it is a monovalent substituent, specific examples may include, although are not limited to, the groups mentioned as examples for R¹³ and R²³ above, as well as protective groups for amino groups (hereinafter also referred to as PG^{a}), and the silicon-containing hydrophobic substituent -TAG(Si), which will be explained later. The protective group for carboxyl groups PG^{b} are not restricted as long as the carboxyl group can be protected so that the carboxyl group concerned does not react in the amidation reaction and can be deprotected and converted to a carboxyl group after the reaction. The details of the protective group for carboxyl groups PG^{b} and the silicon-containing hydrophobic substituent -TAG(Si) will be explained later.

In the compound of formula (R2), the amino group on the left side of the formula may form a salt with a counter acid. In this case, examples of the counter acid include, but are not limited to, aliphatic carboxylic acids with 1 to 5 carbons such as acetic acid and propionic acid; trifluoroacetic acid, hydrochloric acid, hydrobromic acid, sulfuric acid, nitric acid, phosphoric acid, boric acid and sulfonic acid.

Any of the above-mentioned substrate compounds (R1) and (R2) may be used either singly or in combination of any two or more compounds at any ratios.

Some or all of either the substrate compound (R1) or (R2) may be linked or immobilized to a carrier such as a basal plate or resin at any of the substituents. In this case, the type of the carrier such as the basal plate or resin is not limited. Any carriers such as basal plates or resins known so far can be used to the extent that they do not substantially interfere with the amide bond reaction in the production method of the present invention and do not depart from the purpose of the present invention. There is no limitation to the means to link or immobilize the substrate compound to the carrier such as the basal plate or resin, but it may be preferable to form a covalent bond between a substituent of the substrate compound and a substituent present on the basal plate, resin, or other carrier. There are also no limitations on the types of each substituent or the method of forming the covalent bond. Any types of substituents and methods for forming a covalent bond known so far can be used, as long as they do not substantially interfere with the amide bond reaction in the production method of the present invention, and to the extent that they do not depart from the purpose of the present invention. The substrate compound may be linked or immobilized to a basal plate, resin, or other carrier via a covalent bond using a carboxyl or amino group possessed by the substrate compound (other than the carboxyl ester or amino group that is the target of the amide bond reaction formation). Such an embodiment can be regarded similarly to an embodiment in which the carboxyl or amino group possessed by the substrate compound (other than the carboxyl ester or amino group that is the target of the amide bond reaction formation) is protected by introducing a protecting group.

### *Protective group for amino groups:

Various protecting groups for amino groups PG^{a} are known to the art. Examples include monovalent hydrocarbon groups that may have one or more substituents and monovalent heterocyclic groups that may have one or more substituents. Preferred among them include monovalent hydrocarbon groups that may have one or more substituents. However, a linking group may intervene between the hydrocarbon group or heterocyclic group and the nitrogen atom of the amino group it protects. The linking group may be, independently of each other, selected from, although is not limited to, the following groups (where, in the chemical formulae below, A represents, independently of each other, a monovalent hydrocarbon group or heterocyclic group that may have one or more substituents. When two A's are present in the same group, they may be identical to each other or different from each other.).

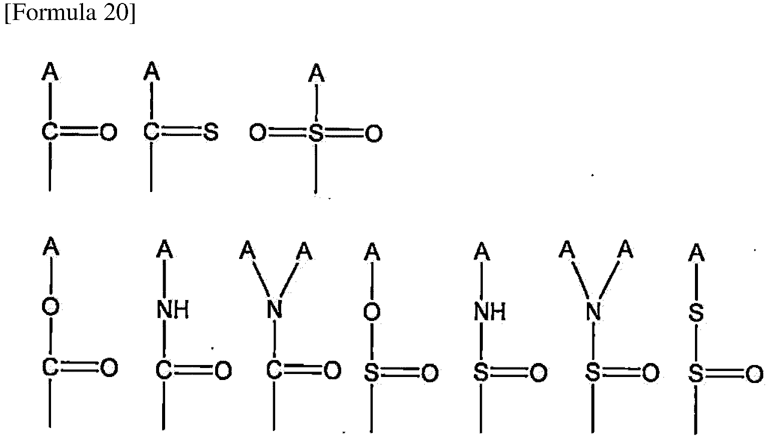

The number of carbons in the protective group may be typically 1 or more, or 3 or more, and typically 20 or less, or 15 or less.

Among them, the amino-protecting group may preferably be one or more selected from the group consisting of monovalent hydrocarbon groups, acyl groups, hydrocarbon oxycarbonyl groups, hydrocarbon sulfonyl groups and amides that may have one or more substituents.

Specific examples of the amino-protective group are listed below. Incidentally, an amino-protective group may be referred to either by the name of the functional group excluding the nitrogen atom of the amino group to which it binds or by the name of the group including the nitrogen atom to which it binds. The following list includes either or both of these names for each protective group.

Examples of unsubstituted or substituted hydrocarbon groups includes: alkyl groups such as methyl group, ethyl group, and propyl group; alkenyl groups such as ethenyl group, propenyl group, and allyl group; alkynyl groups such as propargyl group; cycloalkyl groups such as cyclopropyl group, cyclobutyl group, cyclopentyl group, and cyclohexyl group; aryl groups such as phenyl group, benzyl group, p-methoxybenzyl group, tolyl group, and triphenylmethyl group (Troc group); and substituted hydrocarbon groups such as cyanomethyl group. The number of carbon atoms may typically be 1 or more, or 3 or more, and typically 20 or less, or 15 or less.

Examples of unsubstituted or substituted acyl groups includes: benzoyl group (Bz), o-methoxybenzoyl group, 2,6-dimethoxy benzoyl group, p-methoxybenzoyl group (PMPCO), cinnamoyl group, and phthaloyl group (Phth).

Examples of unsubstituted or substituted hydrocarbon oxycarbonyl groups includes: tert-butoxycarbonyl group (Boc), benzyloxycarbonyl group (Cbz or Z), methoxycarbonyl group, ethoxycarbonyl group, 2-(trimethylsilyl)ethoxycarbonyl group, 2-phenyl ethoxycarbonyl group, 1-(1-adamanthyl)-1-methylethoxycarbonyl group, 1-(3,5-di-t-butylphenyl)-1-methylethoxycarbonyl group, vinyloxycarbonyl group, allyloxycarbonyl group (Alloc), N-hydroxypiperidinyloxycarbonyl group, p-methoxybenzyloxycarbonyl group, p-nitrobenzyloxycarbonyl group, 2-(1,3-dithianyl)methoxycarbonyl, m-nitrophenoxycarbonyl group, 3,5-dimethoxybenzyloxycarbonyl group, o-nitrobenzyloxycarbonyl group, 2,2,2-trichloroethoxycarbonyl group (Troc), and 9-fluorenylmethyloxycarbonyl group (Fmoc).

Examples of unsubstituted or substituted hydrocarbon sulfonyl groups includes: methane sulfonyl group (Ms), toluenesulfonyl group (Ts), and 2- or 4-nitro benzene sulfonyl (Ns) group.

Examples of unsubstituted or substituted amide groups includes: acetamide, o-(benzoyloxymethyl)benzamide, 2-[(t-butyl-diphenyl-siloxy)methyl]benzamide, 2-toluenesulfonamide, 4-toluenesulfonamide, 2-nitro benzene sulfonamide, 4-nitro benzene sulfonamide, tert-butylsulfinyl amide, 4-toluenesulfonamide, 2-(trimethylsilyl)ethane sulfonamide, and benzyl sulfonamide.

In terms of deprotection methods, the protective group may be deprotected by, e.g., at least one of the following methods: deprotection by hydrogenation, deprotection by weak acid, deprotection by fluorine ion, deprotection by one-electron oxidizing agent, deprotection by hydrazine, and deprotection by oxygen.

Preferred examples of the amino protective group include mesyl group (Ms), tert-butoxycarbonyl group (Boc), benzyl group (Bn or Bzl), benzyloxycarbonyl group (Cbz), benzoyl group (Bz), p-methoxybenzyl group (PMB), 2,2,2-trichloroethoxycarbonyl group (Troc), allyloxycarbonyl group (Alloc), 2,4-dinitrophenyl group (2,4-DNP), phthaloyl group (Phth), p-methoxybenzoyl group (PMPCO), cinnamoyl group, toluenesulfonyl group (Ts), 2- or 4-nitrobenzenesulfonyl group (Ns), cyanomethyl group, and 9-fluorenylmethyloxycarbonyl group (Fmoc). These protective groups are preferred because, as mentioned above, they can easily protect the amino group and can be removed under relatively mild conditions.

More preferable examples of the amino protective group include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, cinnamoyl group, 2- or 4-nitrobenzenesulfonyl group (Ns), toluenesulfonyl group (Ts), phthaloyl group (Phth), 2,4-dinitrophenyl group (2,4-DNP), and 9-fluorenylmethyloxycarbonyl group (Fmoc).

### *Protective group for carboxyl groups:

Various protective groups for carboxyl groups are known to the art. Examples include a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Specific examples of protective group for carboxyl groups may include, although are not limited to, the following.

*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Aryl groups such as phenyl, benzyl, tolyl, naphthyl and anthracenyl groups;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
*Silicon-based protective groups such as trimethylsilyl (TMS) group, triethylsilyl (TES) group, triisopropylsilyl (TIPS) group, tri(tert-butyl)silyl (TBS) group, tert-butyldiphenylsilyl (TBDPS) group and tris(trialkylsilyl)silyl group.

### *Silicon-containing hydrophobic substituent -TAG(Si):

One of the main features of the method of the present invention is to satisfy the requirement(s) (I) and/or (ii) below.
(i) T^{a} in formula (R1) is a group represented by -O-C(=O)-TAG(Si).
(ii) T^{b} in formula (R2) is a group represented by -TAG(Si).

In this regard, -TAG(Si) is a silicon-containing hydrophobic substituent having a structure represented by formula (T) below.

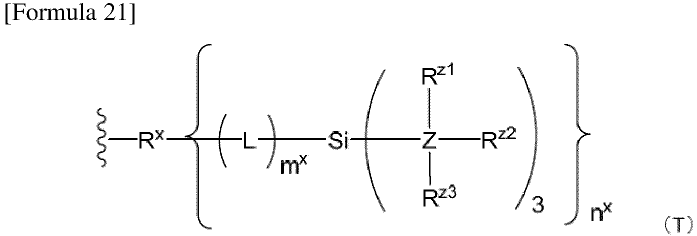

In other words, one of the major features of the method of the present invention is that the amino group on the left side of the amino acid or peptide compound in formula (R1) and/or the carboxyl group on the right side of the amino acid ester or peptide ester compound in formula (R2) are/is protected with the silicon-containing hydrophobic substituent TAG(Si) of formula (T) (or a group containing TAG(Si)) .

In formula (T), R^{x} represents a divalent, trivalent, or tetravalent aliphatic hydrocarbon group, aromatic hydrocarbon group, or heterocyclic group that may have one or more substituents. The divalent, trivalent, or tetravalent aliphatic hydrocarbon group, aromatic hydrocarbon group, or heterocyclic group may be any divalent, trivalent, or tetravalent group derived from a monovalent aliphatic hydrocarbon group, aromatic hydrocarbon group, or heterocyclic group by removing any one, two, or three hydrogen atoms, respectively. If these groups have one or more substituents, they may be selected arbitrarily from those detailed earlier. The number of substituents may be 5, 4, 3, 2, 1, or 0.

The number of carbon atoms in the divalent, trivalent, or tetravalent aliphatic hydrocarbon group, aromatic hydrocarbon group, or heterocyclic group of R^{x} (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

In formula (T), L represents a single bond or a divalent linking group. Examples of the divalent linking group include, although are not limited to, independently of each other, structures selected from the following structures. (The left-right direction in the following chemical formulae is arbitrary; the right-hand bond in the following formulae may be located on the right side of formula (T) and the left-hand bond in the following formulae may be located on the left side of formula (T); alternatively, the right-hand bond in the following formulae may be located on the left side of formula (T) and the left-hand bond in the following formulae may be located on the right side of formula (T).)

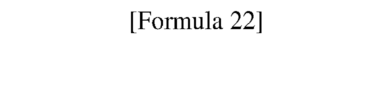

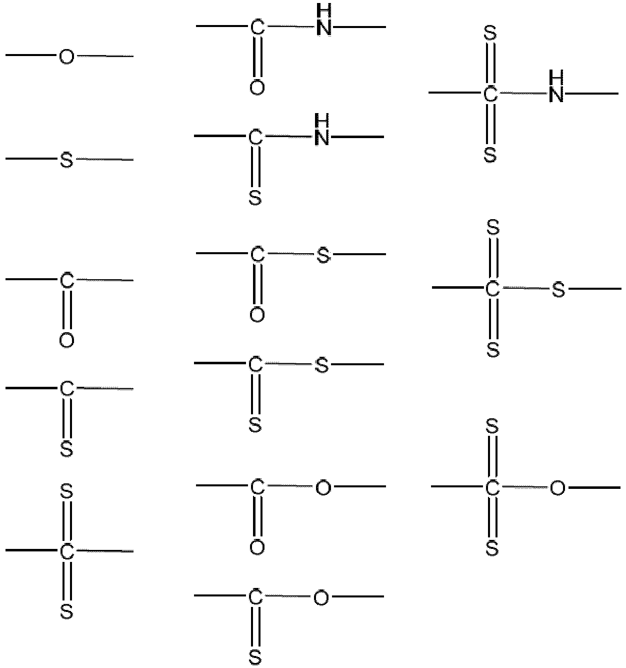

In formula (T), m^{x} represents an integer of 0 or 1 provided that when m^{x} is 0, L is a single bond.

In formula (T), Z represent, independently of each other, a carbon atom (C) or a silicon atom (Si).

In formula (T), R^{z1}, R^{z2}, and R^{z3} represent, independently of each other, a monovalent aliphatic hydrocarbon group, aromatic hydrocarbon group, silicon-containing hydrocarbon group, or heterocyclic group that may have one or more substituents. The monovalent aliphatic and/or aromatic hydrocarbon group (e.g., alkyl group, alkenyl group, alkynyl group, cycloalkyl group, cycloalkenyl group, cycloalkynyl group, aryl group, aryl alkyl group, and alkyl aryl group) and the heterocyclic group have already been explained earlier. Specific examples include, although are not limited to, the following groups.

*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Aryl groups such as phenyl, benzyl, tolyl, naphthyl and anthracenyl groups;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group.

Examples of monovalent silicon-containing hydrocarbon groups are groups derived from monovalent aliphatic hydrocarbon group or aromatic hydrocarbon groups by replacing one or more carbon atoms with silicon atoms.

When R^{z1}, R^{z2}, and/or R^{z3} are/is a monovalent aliphatic hydrocarbon group or aromatic hydrocarbon group, the number of carbon atoms (when it has one or more substituents, including the number of atoms in the substituents) may be, independently of each other, 40 or less, 35 or less, 30 or less, or 25 or less. The lower limit may vary depending on the type of the hydrocarbon group, but it may be 1 or more in the case of alkyl groups, 2 or more or more in the case of alkenyl groups and alkynyl groups, and 3 or more, 4 or more or 5 or more in the case of cycloalkyl groups. When R^{z1}, R^{z2}, and/or R^{z3} are/is a monovalent heterocyclic group, the total number of carbon atoms and hetero atoms (when it has one or more substituents, including the number of atoms in the substituents) may be 40 or less, 35 or less, 30 or less, or 25 or less. The lower limit may vary depending on the type of the hydrocarbon group, but it may be 3 or more, or 4 or more, or 5 or more. Specific examples of the number of these atoms include 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40.

The three substituents R^{z1}, R^{z2}, and R^{z3}, which are bound to Z in formula (T1), may either be the same as each other or different from each other. However, at least one, or 2, or all 3 of these substituents R^{z1}, R^{z2}, and R^{z3} may be a group with a low polarity. Specifically, it may preferably be a monovalent aliphatic hydrocarbon group whose carbon number is 6 or more or an aromatic hydrocarbon group or a heterocyclic group.

The three parenthesized substituents in -Z(R^{z1})(R^{z2})(R^{z3}) may either be the same as each other or different from each other.

In formula (T), n^{x} represents an integer of 1 to 3 corresponding to the number of the substituents parenthesized with { }, provided that when m^{x} is 2 or 3, then the two or three substituents parenthesized with { } in formula (T) may either be the same as each other or different from each other .

When the silicon-containing hydrophobic substituent TAG(Si) of formula (T) protects the terminal amino group on the left side of the amino acid or peptide compound in formula (R1), the -TAG(Si) group is usually bound to the terminal amino radical - N(R¹³)- of the compound of formula (R1) via a carbonyloxy group -C(=O)-O- to form a structure represented by -N(R¹³)-C(=O)-O-TAG(Si). On the other hand, when the silicon-containing hydrophobic substituent TAG(Si) of formula (T) protects the terminal carboxyl group of the amino acid ester or peptide ester compound in formula (R2), the -TAG(Si) group is usually bound directly to terminal carboxyl radical -C(=O)-O- of the compound of formula (R2) to form a structure represented by -C(=O)-O-TAG(Si).

According to the method of the present invention, the silicon-containing hydrophobic substituent -TAG(Si), which has a specific structure explained above, is used to protect the terminal amino group of the amino acid or peptide compound of formula (R1) and/or the terminal carboxyl group of the amino acid or peptide compound of formula (R2), and subjected to extension of a peptide chain via peptide binding reaction using these compounds. This prevents a decrease in the solubility of the peptide chains in organic solvents, which in turn improves the reactivity of the peptide chain and the operability of the purification process. The reasons for this may be considered, but are not bound by theory, as follows. The -TAG(Si) contains a long chain aliphatic group or an aromatic ring or heterocyclic group with a low polarity as any of substituents R^{z1}, R^{z2}, and/or R^{z3}, and also contains a silicon atom, whereby the effect of reducing its polarity may be enhanced. In addition, unlike in the case of the silyl ester described in Non-Patent Literature 7 (Org. Process Res. Dev. 2021, Vol.25, p.2029-2038) cited above, the silicon atom in -TAG(Si) is not bound to the terminal carboxyl group of the amino acid or peptide compound directly, but via R^{x} and L, and this reduces the risk of deprotection of the ester depending on the reaction conditions and allows for application under a wide range of reaction conditions.

Another big advantage provided by the method of the present invention is that the silicon-containing hydrophobic substituent TAG(Si) may also be used for protecting the N terminal amino group of the amino acid or peptide compound of formula (R1). In general, peptide bond forming reactions start from the extension at the N terminal end. For example, in the case of solid phase synthesis methods, since a resin is always bound to the C terminal of a peptide, an extension reaction of the peptide has to be carried out at the N terminal side of the peptide. Also, in the case of liquid phase synthesis methods, the variety of protecting groups that can be used for protecting the N terminal, such as Fmoc, Boc, Cbz, Trt, and Ac, is much lower than the variety of protecting groups that can be used for protecting the C terminal. In addition, these protecting groups are focused on short-chain peptide synthesis and have little effect in reducing the polarity of long-chain peptides. In these terms, the synthesis of N-terminal protecting groups with a view to long-chain peptide synthesis is important, and the silicon-containing hydrophobic substituent TAG(Si) of the present invention is very useful in this respect.

Examples of -TAG(Si) include, although are not limited to, groups that are selected from the groups having the structures represented by formulae (T1), (T2), and (T3).

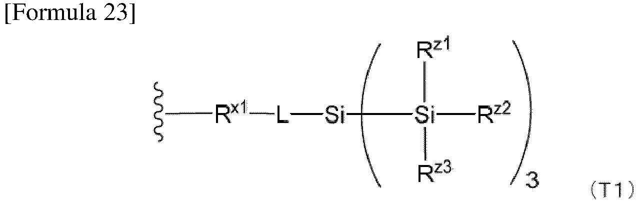

In formula (T1), R^{x1} represents a divalent aliphatic hydrocarbon group, aromatic hydrocarbon group, or heterocyclic group that may have one or more substituents. Examples thereof are the same as those explained above.

In formula (T1), L, R^{z1}, R^{z2}, and R^{z3} represent, independently of each other, the same definitions as those of the same symbols in formula (T). Examples of L include, although are not limited to, oxy group (-O-), carbonyl group (-C(=O)-), carbonyl oxy group (-C(=O)-O-), or oxycarbonyl group (-O-C(=O)-).

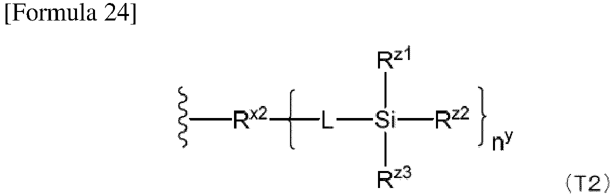

In formula (T2), R^{x2} represents a trivalent or tetravalent aliphatic hydrocarbon group, aromatic hydrocarbon group, or heterocyclic group that may have one or more substituents. Examples thereof are the same as those explained above.

In formula (T2), L, R^{z1}, R^{z2}, and R^{z3} represent, independently of each other, the same definitions as those of the same symbols in formula (T). Preferable examples of L include, although are not limited to, oxy group (-O-), carbonyl group (-C(=O)-), carbonyl oxy group (-C(=O)-O-), or oxycarbonyl group (-O-C(=O)-).

In formula (T2), n^{y} represents an integer of 2 or 3 corresponding to the number of the substituents parenthesized with { }, provided that the two or three substituents parenthesized with { } in formula (T2) may either be the same as each other or different from each other.

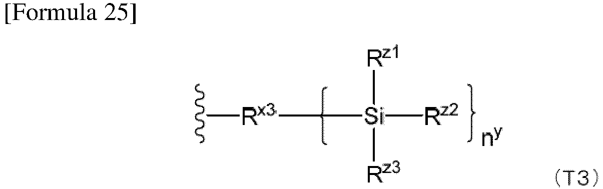

In formula (T3), R^{x3} represents a trivalent or tetravalent aliphatic hydrocarbon group, aromatic hydrocarbon group, or heterocyclic group that may have one or more substituents. Examples thereof are the same as those explained above. Preferable examples of R^{x3} include trivalent or tetravalent groups derived from monovalent aromatic hydrocarbon groups (e.g., aryl group, alkylaryl group, and arylalkyl group) or heterocyclic groups by removing 2 or 3 hydrogen atoms. Specific examples thereof are the same as those mentioned above.

In formula (T3), R^{z1}, R^{z2}, and R^{z3} represent, independently of each other, the same definitions as those of the same symbols in formula (T).

In formula (T3), n^{y} represents an integer of 2 or 3 corresponding to the number of the substituents parenthesized with { }, provided that the two or three substituents parenthesized with { } in formula (T3) may either be the same as each other or different from each other.

Specific examples of the silicon-containing hydrophobic substituents represented by formula (T1), (T2), or (T3) include, although are not limited to, TAG1(Si), TAG2(Si), and TAG3(Si) as demonstrated in Examples 1 to 3 below.

The method for protecting the terminal amino group of the amino acid or peptide compound of formula (R1) or the terminal carboxyl group of the amino acid or peptide compound of formula (R2) with the silicon-containing hydrophobic substituent TAG(Si) 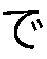 protection is not particularly limited, but may be carried out by any known methods. For example, it may be carried out by preparing a alcohol compound having the structure represented by formula (T0) (hereinafter also referred to as "TAG(Si)-OH"), in which the silicon-containing hydrophobic substituent TAG(Si) is bound to a hydroxyl group (OH), and then binding the alcohol compound TAG(Si)-OH to the terminal amino group or the terminal carboxyl group of the amino acid or peptide compound.

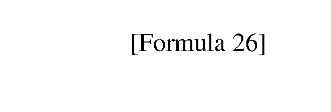

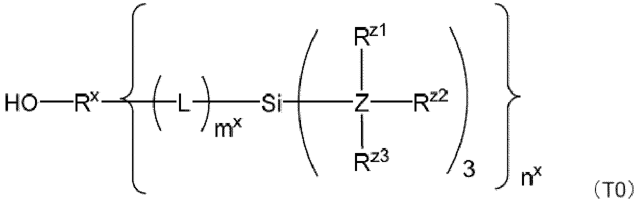

The synthesis of the alcohol compound TAG(Si)-OH of formula (T0) can a person skilled in the art can be carried out by using or combining any known methods depending on the structure of the silicon-containing hydrophobic substituent TAG(Si).

For example, the alcohol compound TAG1(Si)-OH, which has TAG1(Si) as the silicon-containing hydrophobic substituent TAG(Si), can be synthesized by reacting an appropriate combination of a mono-substituted trichlorosilane compound and a tri-substituted chlorosilane compound to form a basic skeleton having the super silyl structure Si(Si)₃, and then binding an appropriate linker to this skeleton by forming a bond between a carbonic acid and a silicon.

The alcohol compound TAG2(Si)-OH, which has TAG2(Si) as the silicon-containing hydrophobic substituent TAG(Si), can be synthesized by reacting an appropriate combination of an alcohol and a tri-substituted chlorosilane compound to form its basic skeleton.

The alcohol compound TAG3(Si)-OH, which has TAG3(Si) as the silicon-containing hydrophobic substituent TAG(Si), can be synthesized by reacting an appropriate combination of an aromatic compound substituted with a bromine and a tri-substituted chlorosilane compound to form its basic skeleton.

The alcohol compound TAG(Si)-OH of formula (T0) can be bound to a terminal amino group of a substrate compound (e.g., an amino acid or peptide compound) by reacting both compounds under basic conditions using a known carbamating reagent such as triphosgene.

The alcohol compound TAG(Si)-OH of formula (T0) can be bound to a terminal carboxyl group of a substrate compound (e.g., an amino acid or peptide compound) by reacting both compounds in the presence of a known condensing agent such as DCC (N,N'-dicyclohexylcarbodiimide) or DMAP (4-dimethylaminopyridine).

Amino acid or peptide compounds of formulae (R1) and (R2) and peptide compounds of formula (P1), which each have a silicon-containing hydrophobic substituent TAG(Si) on the terminal amino group and/or the terminal carboxyl group thereof, are all novel compounds and are subject to the present invention.

### *Silane compound:

In the production method (1) of the present invention, a silane compound may be coexisted in the reaction system. Carrying out the reaction with a silane compound in the reaction system may result in various advantages such as improved reaction yield and stereo selectivity.

Examples of silane compounds include: various tris{halo-(preferably fluorine-)substituted alkyl}silanes such as HSi(OCH(CF₃)₂)₃, HSi(OCH₂CF₃)₃, HSi(OCH₂CF₂CF₂H)₃, HSi(OCH₂CF₂CF₂CF₂CF₂H)₃; as well as trimethylsilyl trifluoromethanesulfonate(TMS-OTf), 1-(trimethylsilyl)imidazole (TMSIM), dimethyl ethylsilyl imidazole (DMESI), dimethyl isopropylsilyl imidazole (DMIPSI), 1-(tert-butyl dimethylsilyl)imidazole (TBSIM), 1-(trimethylsilyl)triazole, 1-(tert-butyl dimethylsilyl)triazole, dimethylsilyl imidazole, dimethylsilyl (2-methyl)imidazole, trimethyl bromosilane (TMBS), trimethyl chlorosilane (TMCS), N-methyl-Ntrimethylsilyl trifluoroacetamide (MSTFA), N,O-bis(trimethylsilyl)trifluoroacetamide (BSTFA), N,O-bis(trimethylsilyl)acetamide (BSA), N-(trimethylsilyl)dimethyl amine (TMSDMA), N-(tert-butyl dimethylsilyl)-N-methyl trifluoroacetamide (MTBSTFA), and hexamethyldisilazane (HMDS). Any one of these may be used alone, or two or more may be used together in any combination and ratio.

### *Lewis acid catalyst:

In the production method (1) of the present invention, the reaction system may also contain a Lewis acid catalyst. Carrying out the reaction with a Lewis acid catalyst in the reaction system may lead to various advantages, such as improved reaction yield and stereoselectivity. On the other hand, however, when a Lewis acid catalyst is used, it may be necessary to separate and remove the Lewis acid catalyst from the reaction product. Therefore, it is preferable to determine whether to use a Lewis acid catalyst taking into consideration the purpose of using the production method according to the present invention.

When a Lewis acid catalyst is used in the production method of the present invention, the type of catalyst is not limited, but it may preferably be a metal compound that functions as a Lewis acid. Examples of metal elements constituting the metal compound include various metals belonging to groups 2 through 15 of the periodic table. Examples of such metal elements include boron, magnesium, gallium, indium, silicon, calcium, lead, bismuth, mercury, transition metals, and lanthanoid elements. Examples of transition metals include scandium, titanium, vanadium, chromium, manganese, iron, cobalt, nickel, copper, zinc, yttrium, zirconium, niobium, molybdenum, technetium, ruthenium, rhodium, palladium, tin, silver, cadmium, hafnium, tantalum, tungsten, rhenium, osmium, iridium, platinum, gold, and thallium. Examples of lanthanoid elements include lanthanum, cerium, neodymium, samarium, europium, gadolinium, holmium, erbium, thulium, ytterbium. From the viewpoint of excellent reaction acceleration and highly stereoselective production of amide compounds, the metal element may preferably be one or more selected from titanium, zirconium, hafnium, tantalum, niobium, boron, vanadium, tungsten, neodymium, iron, lead, cobalt, copper, silver, palladium, tin, and thallium, more preferably one or more selected from titanium, zirconium, hafnium, tantalum, and niobium. The metal compound may contain one, two or more metal atoms. If the metal compound contains two or more metal atoms, the two or more metal atoms may be either of the same metal element or of different metal elements.

Ligands constituting the metal compound may be selected according to the type of the metal element. Examples of ligands include: substituted or unsubstituted linear- or branched-chain alkoxy groups containing 1 to 10 carbon atoms, such as methoxy group, ethoxy group, propoxy group, butoxy group, trifluoroethoxy group, and trichloroethoxy group; halogen atoms such as fluorine atom, chlorine atom, bromine atom, iodine atom; aryloxy groups having 1 to 10 carbon atoms; acetylacetonate group (acac), acetoxy group (AcO), trifluoromethane sulfonate group (TfO); substituted or unsubstituted linear- or branched-chain alkyl groups having 1 to 10 carbon atoms; phenyl group, oxygen atom, sulfur atom, group -SR (where R represents a substituent exemplified by substituted or unsubstituted hydrocarbon groups containing 1 to 20 carbon atoms), group -NRR' (where R and R', independently of each other, represent a hydrogen atom or a substituent exemplified by substituted or unsubstituted hydrocarbon groups containing 1 to 20 carbon atoms), and cyclopentadienyl (Cp) group.

Preferred metal compounds among these are titanium compounds, zirconium compounds, hafnium compounds, tantalum compounds, or niobium compounds. Examples of these metal compounds are indicated below. Any one of these may be used alone, or two or more may be used together in any combination and ratio.

Examples of titanium compounds include those represented by TiX¹₄ (where 4 X¹'s, independently of each other, represent any of the ligands exemplified above, provided that 4 X¹'s may be the same type of ligand or different from each other.). When X¹ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X¹ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X¹ is a halogen atom, it may preferably be a chlorine atom or a bromine atom. Preferred examples of titanium compounds include Ti(OMe)₄, Ti(OEt)₄, Ti(OPr)₄, Ti(Oi-Pr)₄, Ti(OBu)₄, Ti(Ot-Bu)₄, Ti(OCH₂CH(Et)Bu)₄, CpTiCl₃, Cp₂TiCl₂, Cp₂Ti(OTf)₂, (i-PrO)₂TiCl₂, and (i-PrO)₃TiCl.

Examples of zirconium compounds include those represented by ZrX²₄ (where 4 X²'s, independently of each other, represent any of the ligands exemplified above, provided that 4 X²'s may be the same type of ligand or different from each other.). When X² is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X² is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X² is a halogen atom, it may preferably be a chlorine atom or a bromine atom. Preferred examples of zirconium compounds include Zr(OMe)₄, Zr(OEt)₄, Zr(OPr)₄, Zr(Oi-Pr)₄, Zr(OBu)₄, Zr(Ot-Bu)₄, Zr(OCH₂CH(Et)Bu)₄, CpZrCl₃, Cp₂ZrCl₂, Cp₂Zr(OTf)₂, (i-PrO)₂ZrCl₂, and (i-PrO)₃ZrCl.

Examples of hafnium compounds include those represented by HfX³₄ (where 4 X³'s, independently of each other, represent any of the ligands exemplified above, provided that 4 X³'s may be the same type of ligand or different from each other.). When X³ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X³ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X³ is a halogen atom, it may preferably be a chlorine atom or a bromine atom. Preferred examples of hafnium compounds include HfCp₂Cl₂, HfCpCl₃, and HfCl₄.

Examples of tantalum compounds include those represented by TaX⁴₅ (where 5 X⁴'s, independently of each other, represent any of the ligands exemplified above, provided that 5 X⁴'s may be the same type of ligand or different from each other.). When X⁴ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁴ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁴ is a halogen atom, it may preferably be a chlorine atom or a bromine atom. Preferred examples of tantalum compounds include tantalum alkoxide compounds (e.g., compounds in which X⁴ is an alkoxy group) such as Ta(OMe)₅, Ta(OEt)₅, Ta(OBu)₅, Ta(NMe₂)₅, Ta(acac)(OEt)₄, TaCl₅, TaCl₄(THF), and TaBrs. Other examples are those in which X⁴ is an oxygen, such as Ta₂O₅.

Examples of niobium compounds include those represented by NbX⁵₅ (where 5 X⁵'s, independently of each other, represent any of the ligands exemplified above, provided that 5 X⁵'s may be the same type of ligand or different from each other.). When X⁵ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁵ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁵ is a halogen atom, it may preferably be a chlorine atom or a bromine atom. Preferred examples of niobium compounds include niobium alkoxide compounds (e.g., compounds in which X⁵ is an alkoxy group) such as NbCl₄(THF), NbCl₅, Nb(OMe)₅, and Nb(OEt)₅. Other examples are those in which X⁵ is an oxygen, such as Nb₂O₅.

The Lewis acid catalyst may be loaded on a carrier. There are no particular restrictions on the carrier on which the Lewis acid catalyst is to be loaded, and any known carrier can be used. Also, any known method can be used to load the Lewis acid catalyst on the carrier.

### *Other ingredients:

In the production method (1) of the present invention, any other ingredients may coexist in the system in addition to the substrate compounds, i.e., the amino acid or peptide compound of formula (R1) and the amino acid ester or peptide ester compound of formula (R2); the amidation reaction agent, i.e., the aluminum compounds represented by formula (A); and a silane compound and/or a Lewis acid catalyst, which may optionally be used. Examples of other ingredients may include, although are not limited to, conventional catalysts (other than Lewis acid catalysts) that can be used for an amidation reaction, bases, phosphorus compounds, and solvents. Any one of these may be used alone, or two or more may be used together in any combination and ratio.

Examples of catalysts (other than Lewis acid catalysts) include methylaluminum bis(4-bromo-2,6-di-tert-butylphenoxyde (MABR), trimethylsilyl trifluoromethanesulfonate (TMS-OTf), and methylaluminum bis(2,6-di-tert-butylphenoxyde (MAD). Any one of these may be used alone, or two or more may be used together in any combination and ratio.

The type of base is not restricted, and any base that is known to improve reaction efficiency can be used. Examples of such bases include amines having 1 to 4 linear or branched-chain alkyl groups with 1 to 10 carbons, such as tetrabutylammonium fluoride (TBAF), triethylamine (Et₃N), diisopropylamine (i-Pr₂NH), and diisopropylethylamine (i-Pr₂EtN), as well as inorganic bases such as cesium fluoride. Any one of these may be used alone, or two or more may be used together in any combination and ratio.

Examples of phosphorus compounds include: phosphine compounds such as trimethyl phosphine, triethyl phosphine, tripropyl phosphine, trimethyloxyphosphine, triethyloxyphosphine, tripropyloxyphosphine, triphenyl phosphine, trinaphthyl phosphine, triphenyloxyphosphine, tris(4-methylphenyl)phosphine, tris(4-methoxy phenyl)phosphine, tris(4-fluorophenyl)phosphine, tris(4-methylphenyloxy)phosphine, tris(4-methoxy phenyloxy)phosphine, and tris(4-fluorophenyloxy)phosphine; phosphate compounds such as trimethyl phosphate, triethyl phosphate, tripropyl phosphate, trimethyloxyphosphate, triethyloxyphosphate, tripropyloxyphosphate, triphenyl phosphate, trinaphthyl phosphate, triphenyloxyphosphate, tris(4-methylphenyl)phosphate, tris(4-methoxy phenyl)phosphate, tris(4-fluorophenyl)phosphate, tris(4-methylphenyloxy)phosphate, tris(4-methoxy phenyloxy)phosphate, and tris(4-fluorophenyloxy)phosphate; multivalent phosphine compounds or multivalent phosphate compounds such as 2,2'-bis(diphenylphosphino)-1,1'-binaphthyl (BINAP) and 5,5'-bis(diphenylphosphino)-4,4'-bi-1,3-benzodioxole (SEGPHOS). Any one of these may be used alone, or two or more may be used together in any combination and ratio.

From the viewpoint of increasing reaction efficiency, the reaction may be carried out in a solvent. Examples of solvents include, although are not limited to, aqueous solvents and organic solvents. Examples of organic solvents may include, although are not limited to: aromatic hydrocarbons such as toluene and xylene; eters such as pentane, petroleum ether, tetrahydrofuran (THF), 1-methyl tetrahydrofuran (1-MeTHF), diisopropyl ether (i-Pr₂O), diethyl ether (Et₂O), and cyclopentylmethyl ether (CPME); nitrogen-containing organic solvents acetonitrile (MeCN); chlorine-containing organic solvents such as dichloromethane (DCM); esters such as ethyl acetate (AcOEt); and organic acids such as acetic acids. Any one of these solvents may be used alone, or two or more may be used together in any combination and ratio.

### *Reaction procedure:

In the production method (1) of the present invention, the substrate compounds, i.e., the amino acid or peptide compound of formula (R1) and the amino acid ester or peptide ester compound of formula (R2), may be mixed with the amidation reaction agent, i.e., the aluminum compounds represented by formula (A), to cause a reaction. When a silane compound, a Lewis acid catalyst, and/or other ingredients (e.g., a catalyst other than Lewis acid catalysts, a base, and/or a phosphorus compound) are used as optional ingredients, they may be mixed with the substrate compounds and the amidation reaction agent. When a solvent is optionally used, the above ingredients may be added to the solvent and mixed in the solvent.

For each of the above ingredients, the entire amount may be added to the system all at once, separately in several portions, or continuously in small amounts.

### *Ratios of the amounts of the ingredients used:

In the production method (1) of the present invention, the amount of each component used is not limited, but may preferably be as follows.

The amount ratio of the amino acid or peptide compound of formula (R1) to the amino acid ester or peptide ester compound of formula (R2) is not restricted, but relative to 1 mol of the compound of formula (R1), the compound of formula (R2) may be used in an amount within a range of 0.1 mol or more, or 0.2 mol or more, or 0.3 mol or more, or 0.4 mol or more, or 0.5 mol or more, and 20 mol or less, or 10 mol or less, or 5 mol or less, or 4 mol or less, or 3 mol or less. It may be preferably to use the compound of formula (R2) in a larger amount than the amount of the compound of formula (R1), from the viewpoint of increasing the reaction efficiency. Specifically, relative to 1 mol of the compound of formula (R1), the compound of formula (R2) may be used in an amount of about 2 mol. Needless to say, it is necessary to use at least 1 mol each of the compound of formula (R1) and the compound of formula (R2) as substrates for the target production volume of the compound of formula (P1) to be manufactured.

The amount of the aluminum compounds represented by formula (A) to be used is not restricted as long as it can induce the formation reaction of a peptide compound of formula (P1) from an amino acid or peptide compound of formula (R1) and an amino acid ester or peptide ester compound of formula (R2) through the practice of the production method of the present invention. For example, relative to 1 mol of the compound of formula (R1), the aluminum compound represented by formula (A) may be used in an amount within a range of 0.1 mol or more, or 0.2 mol or more, or 0.3 mol or more, or 0.4 mol or more, or 0.5 mol or more, and 20 mol or less, or 10 mol or less, or 5 mol or less, or 4 mol or less, or 3 mol or less. When two or more aluminum compounds represented by formula (A) are used in combination, the total amount of the two or more aluminum compounds of formula (A) may satisfy the amount ranges mentioned above.

When a silane compound is used, the amount used is not restricted, but when the amount of the compound of formula (R1) is 100 mol %, the silane compound may be used in an amount of 0.1 mol % or more, or 0.2 mol % or more, or 0.3 mol % or more, or 50 mol % or less, or 30 mol % or less, or 2 0 mol % or less, or 15 mol % or less.

When a Lewis acid catalyst is used, the amount used is not restricted, but when the amount of the compound of formula (R1) is 100 mol%, the Lewis acid catalyst may be used in an amount of 0.1 mol% or more, or 0.2 mol% or more, or 0.3 mol% or more, or 50 mol% or less, or 30 mol% or less, or 20 mol% or less, or 15 mol% or less.

When other optional ingredients are used, their amounts should be adjusted accordingly, referring, for example, to the previous findings of the inventor and others in previous patents (Patent Literatures 1 to 6).

### *Reaction conditions:

In the production method (1) of the present invention, the reaction conditions are not restricted as long as the reaction proceeds, but may be exemplified as follows.

The reaction temperature is not restricted as long as the reaction proceeds, but may be 0 °C or more, or 10 °C or more, or 20 °C or more, and 100 °C or less, or 80 °C or less, or 60 °C or less.

The reaction pressure is not restricted as long as the reaction proceeds, and the reaction may be carried out under reduced, normal, or pressurized pressure, but may typically be carried out under normal pressure.

The reaction atmosphere is also not limited as long as the reaction proceeds, but the reaction may be carried out under an atmosphere of inert gas such as argon or nitrogen.

The reaction time is also not limited as long as the reaction proceeds. However, in order for the reaction to proceed sufficiently and efficiently, the reaction time may be 10 minutes or more, or 20 minutes or more, or 30 minutes or more, and for 80 hours or less, or for 60 hours or less, or for 50 hours or less.

The production method (1) of the present invention may be carried out in a sequential manner (batch) or in a continuous manner (flow). Details of specific procedures for implementing a sequential method (batch method) and a continuous method (flow method) are well known in the art.

### *Post-treatments (e.g., purification and recovery):

The peptide compound (P1) produced by the production method (1) of the present invention may be subjected to various post-treatments. For example, the peptide compound (P1) produced can be isolated and purified according to conventional methods such as column chromatography and recrystallization. In addition, when the peptide compound (P1) produced has an amino group and/or a carboxyl group each protected by a protective group, deprotection can be performed according to the method described below. The generated peptide compound (P1) may be subjected, either directly or after isolation and purification, to the later stage process of the production method (2) of the present invention described below to produce a polypeptide with further elongated amino acid residues.

### [III. Others]

The polypeptide compound of formula (P1) or formula (P2) obtained by the production method mentioned above may be subjected to various post-treatments.

For example, the polypeptide compound of formula (P1) or formula (P2) obtained by the production method mentioned above can be isolated and purified according to conventional methods such as column chromatography and recrystallization.

In addition, the polypeptide compound of formula (P1) or formula (P2) obtained by the production method mentioned above can be subjected to deprotection of the amino group protected by the protecting group. The method of deprotecting the protected amino group is not particularly restricted, and various methods can be used depending on the type of the protecting group. Examples include deprotection by hydrogenation, deprotection by weak acids, deprotection by fluorine ions, deprotection by one-electron oxidants, deprotection by hydrazine, and deprotection by oxygen. The deprotection by hydrogenation may be carried out by, e.g.; (a) a method of causing deprotection in the presence of hydrogen gas using a metal catalyst such as palladium, palladium-carbon, palladium hydroxide, palladium-carbon hydroxide, etc., as a reduction catalyst; and (b) a method of causing deprotection in the presence of a metal catalyst such as palladium, palladium-carbon, palladium hydroxide, palladium-carbon hydroxide, etc., using a hydrotreating reductant such as sodium borohydride, lithium aluminum hydride, lithium borohydride, diborane, etc.

Likewise, the polypeptide compound of formula (P1) or formula (P2) obtained by the production method mentioned above can be subjected to deprotection of the carboxyl group protected by the protecting group. The method of deprotecting the protected carboxyl group is not particularly restricted, and various methods can be used depending on the type of the protecting group. Examples include deprotection by hydrogenation, deprotection by bases, and deprotection by weak acids. In the case of deprotection with a base, a strong base such as lithium hydroxide, sodium hydroxide, potassium hydroxide, etc. can be used.

In addition, the polypeptide compound of formula (P1) or formula (P2) obtained by the production method mentioned above may be used (after deprotection and/or replacement of substituents if necessary) as a peptide compound of formula (R1) again for the production method (1) or (2) of the present invention. Alternatively, the polypeptide compound of formula (P1) or formula (P2) obtained by the production method mentioned above may be subjected (after deprotection and/or replacement of substituents if necessary) to other conventionally known amidation or peptide production methods. Thus, a polypeptide compound of formula (P1) or formula (P2) can be linked to other amino acids or peptides by amide bonding to elongate the amino acid residues and synthesize larger polypeptides. Polypeptides of any number of amino acid residues and amino acid sequences can in principle be synthesized by sequentially repeating these steps.

The present inventors have filed the following prior patent applications relating to amidation reactions for linking amino acids or peptides and methods for producing polypeptides thereby. It is possible to perform the various polypeptide production methods of the present invention in combination with the amidation reactions and polypeptide production methods described in these earlier patent applications as appropriate and/or to modify the amidation reactions and polypeptide production methods described in these prior patent applications as appropriate, taking into account the conditions of these prior patent applications. The descriptions of these earlier patent applications are incorporated herein by reference in their entirety.
(1) WO2017/204144 A (May 22, 2017)
(2) WO2018/199146 A (April 25, 2018)
(3) WO2018/199147 A (April 25, 2018)
(4) WO2019/208731 A (April 25, 2019)
(5) WO2021/085635 A (October 30, 2020)
(6) WO2021/085636 A (October 30, 2020)
(7) WO2021/149814 A (January 22, 2021)

The silicon-containing hydrophobic substituent TAG(Si) to be used in the present invention is applicable not only for the protection of amino acids and peptides but also to other various organic molecules. Examples of such organic molecules include, although are not limited to, saccharides (e.g., various monosaccharides, disaccharides, and polysaccharides), lipids (e.g., fatty acids and triacyl glycerols), composite proteins (e.g., glycoproteins and lipoproteins), and composite lipids (e.g., sugar lipids, sphingolipids, and phospholipids). It is possible to protect any carboxyl group and/or amino group of such organic molecules by binding the silicon-containing hydrophobic substituent TAG(Si) by the methods mentioned above or similar methods. Preferred among these are various organic molecules with a low solubility to organic solvents, such as saccharides, glycoproteins, and lipoproteins, since the silicon-containing hydrophobic substituent TAG(Si) of the present invention can be used to protect the carboxyl group and/or the amino group thereof to thereby increase their solubility to organic solvents. This facilitates the use of these organic molecules in various reactions, particularly flow reactions. In addition, the silicon-containing hydrophobic substituent TAG(Si) can be used to protect either the carboxyl group or the amino group, which provides the advantage of being extremely flexible, as it can protect the group that does not interfere with the reaction depending on the type of reaction desired. Such application of the silicon-containing hydrophobic substituent TAG(Si) of the invention to organic molecules other than amino acids or peptides is also included in the subject of the present invention.

The aforementioned alcohol compound, in which the silicon-containing hydrophobic substituent TAG (Si) is bound to a hydroxyl group (OH), i.e., the alcohol compounds having the structure of formula (T0) below, is also included in the subject of the present invention.
[Formula 27]

TAG(Si)-OH (T0)

Furthermore, the compounds produced by such protection reactions, in which the carboxyl and/or amino groups of various organic molecules are protected by the aforementioned silicon-containing hydrophobic substituents TAG(Si), i.e., the compounds represented by the following formulae (C1) or (C2), are also included in the subject of the present invention.

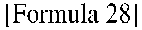

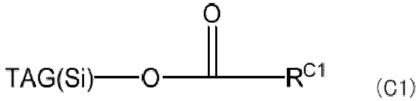

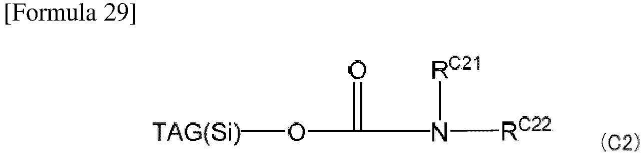

R^{C1} in formula (C1) and R^{C21} and R^{C22} in formula (C2) represent, independently of each other, a monovalent group. Specific examples of R^{C1}, R^{C21}, and R^{C22} include various monovalent substituents mentioned above, such as halogen atoms, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyl oxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyl oxy group, heterocyclic oxy group, heterocyclic carbonyl group, heterocyclic oxycarbonyl group, heterocyclic carbonyl oxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyl oxy group. Other specific examples of R^{C1}, R^{C21}, and R^{C22} include monovalent groups derived from various organic molecules mentioned above, such as saccharides (e.g., various monosaccharides, disaccharides, and polysaccharides), lipids (e.g., fatty acids, triacyl glycerols), composite proteins (e.g., glycoproteins and lipoproteins), and composite lipids (e.g., sugar lipids, sphingolipids and phospholipids).

R^{C1} in formula (C1) may preferably be a monovalent group derived from an organic molecule having a carboxyl group by removing the carboxyl group. In this case, the compound of formula (C1) corresponds to a compound derived from such an organic molecule by protecting the carboxyl group with the silicon-containing hydrophobic substituent TAG(Si). The compound of formula (C1) can be obtained by reacting the organic molecule with an alcohol compound having the structure of formula (T0) by the known methods described above.

In addition, at least either of R^{C21} and R^{C22} in formula (C2) may preferably be a monovalent group derived from an organic molecule having an amino group by removing the amino group. In this case, the compound of formula (C2) corresponds to a compound derived from such an organic molecule by protecting the amino group with the silicon-containing hydrophobic substituent TAG(Si). The compound of formula (C2) can be obtained by reacting the organic molecule with an alcohol compound having the structure of formula (T0) by the known methods described above.

### EXAMPLES

The present invention will be described in more detail in accordance with the examples below, but these examples are only shown for convenience of explanation and the present invention is not limited to these examples in any way. In the following descriptions and reaction formulae, "TAG1(Si)", "TAG2(Si)" and "TAG1(Si)" may be abbreviated simply as "TAG1", "TAG2" and "TAG3", respectively, for the sake of simplicity.

### [Examples 1: Silicon-containing hydrophobic substituent TAG1(Si)]

### *Synthesis of TAG1(Si) reagent (TAG1(Si)-OH):

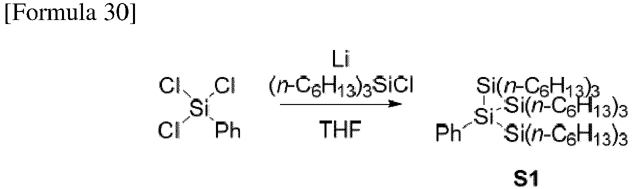

A small piece of lithium (388.6mg, 56.0mmol, 8.0 equivalents) was added dropwise to THF (50mL) at room temperature under a nitrogen atmosphere. The solution was then combined with trichlorophenyl silane (7.0mmol, 1.0 equivalents) and trihexyl chlorosilane (22.4mmol, 3.2 equivalents), and agitated at room temperature for two days. The reaction solution was partitioned via extraction with hexane and water, and the organic phase was dried over sodium sulfate, and purified by silica gel chromatography (hexane) to give the desired supersilyl compound of formula S1 in 87% yield (5.84 g).

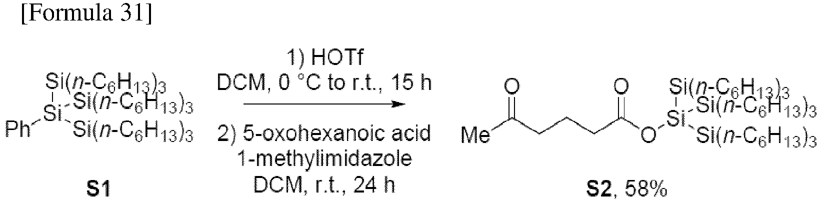

The supersilyl compound of formula S1 was added to dichloromethane (9mL) under a nitrogen atmosphere at 0 °C, to which trifluoromethane sulfonic acid (3.0mmol, 1.5 equivalents) was added at room temperature. After 15 hours, a solution of 5-oxohexanoic acid (2.0 mmol, 1.0 equivalent) and 1-methylimidazole (3.4 mmol, 1.7 equivalents) in dichloromethane (4 mL) was slowly added at room temperature. After 24 hours, the reaction solution was diluted with hexane, filtered, and the solvent was removed using an evaporator. Subsequent purification by silica gel chromatography (hexane/ethyl acetate = 80/1) gave the desired super silyl-containing compound TAG1(Si) of formula S2 in 58% yield (1.16 g).

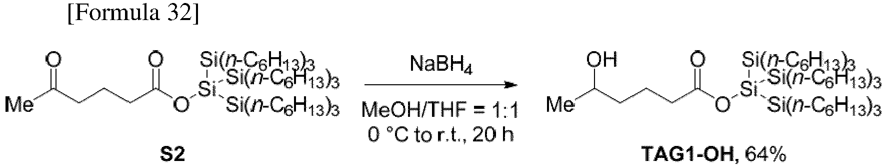

The supersilyl-containing compound TAG1(Si) of formula S2 (1.16 mmol, 1.0 equivalent) was added to a methanol/tetrahydrofuran mixture dropwise at room temperature under a nitrogen atmosphere; the mixture was cooled to 0°C, and sodium borohydride (2.3 mmol, 2.0 equivalent) was added dropwise. The mixture was brought to room temperature, agitated for 20 hours, then combined with saturated sodium hydrogean chloride, and partitioned via extraction with ethyl acetate. The organic phase was dried with sodium sulfate, and the solvent was removed using an evaporator. Purification by silica gel chromatography (hexane/ethyl acetate = 5011) gave the desired alcohol compound TAG1(Si)-OH in 64% yield (744.9 mg).

### *Synthesis of an amino acid whose terminal carboxyl was protected with TAG1(Si) (1):

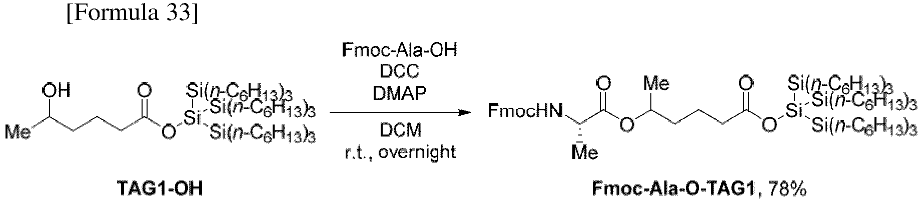

The alcohol compound TAG1(Si)-OH (0.15 mmol, 1.0 equivalent) was added dropwise to dichloromethane (1.5 mL) at room temperature. Fmoc-Ala-OH (0.45 mmol, 3.0 equivalents) was added to the reaction solution, followed by the addition of DMAP (0.18 mmol, 1.2 equivalents) and DCC (0.45 mmol, 3.0 equivalents). After agitating at room temperature overnight, the product was filtered, and the solvent was removed using an evaporator. Purification by silica gel chromatography (hexane/ethyl acetate = 40/1) gave the desired Fmoc-Ala-O-TAG1(Si) in 78% yield (153.4 mg). The characterization data of the obtained Fmoc-Ala-O-TAG1(Si) are indicated below.

Rf = 0.57 (hexanes/EtOAc = 5:1).
[α]_{D}²¹ = -9.43 (c 1.06, CHCl₃).
¹H NMR (400 MHz, CDCl₃) δ 7.77 (d, J = 7.5 Hz, 2H), 7.68 - 7.54 (m, 2H), 7.45 - 7.35 (m, 2H), 7.35 - 7.27 (m, 2H), 5.49 - 5.35 (m, 1H), 5.03 - 4.89 (m, 1H), 4.52 - 4.31 (m, 3H), 4.29 - 4.18 (m, 1H), 2.36 - 2.10 (m, 2H), 1.72 - 1.55 (m, 4H), 1.51 - 1.21 (m, 78H), 0.99 - 0.84 (m, 27H), 0.83 - 0.52 (m, 18H).
¹³C NMR (100 MHz, CDCl₃, two isomers) δ 174.5, 174.4, 172.6, 172.5, 155.55, 155.49, 143.9, 143.8, 141.3(2C), 127.7(2C), 127.0(2C), 125.1(2C), 119.9(2C), 72.1(2C), 67.0(2C), 49.74, 49.69, 47.2(2C), 35.6(2C), 35.4, 35.3, 33.9(2C), 31.6(2C), 25.0(2C), 23.1(2C), 22.7(2C), 21.04, 20.99, 18.92, 18.87, 14.1(4C).
IR (neat) 2955, 2920, 2871, 2854, 1727, 1704, 1451, 1378, 1334, 1252, 1205, 1182, 1132, 1100, 1071 cm⁻¹.
HRMS (ESI) Calcd for C₇₈H₁₄₃NO₆Si₄Na [M+Na]⁺: 1324.9890, Found: 1324.9890.

### *Peptide chain elongation reaction using an amino acid whose terminal carboxyl was protected with TAG1 (Si):

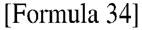

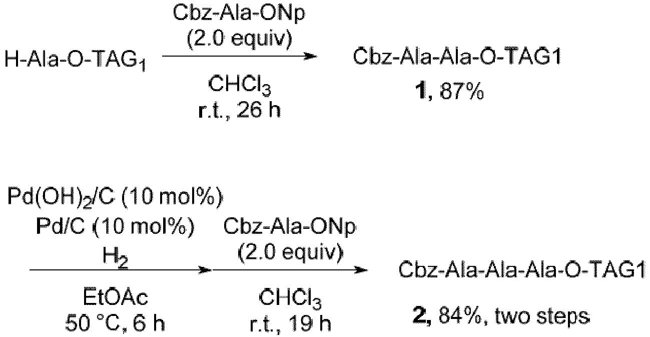

A sequential elongation reaction was examined using an amino acid whose terminal carboxyl group was protected with TAG1(Si). Specifically, the reaction was carried out as follows.

(1) A 6-mL vial was filled with a stirrer, H-Ala-O-TAG1 (0.09 mmol), chloroform (0.2 mL), and Cbz-Ala-ONp (2.0 equivalents; 0.18 mmol), sealed, and agitated at room temperature. After 26 hours, a fractional extraction was performed with dichloromethane/water bicarbonate, the oil phase was dried over sodium sulfate and the solvent was removed using an evaporator. The residue was isolated by column chromatography (hexane/ethyl acetate (EtOAc) = 7:1) to afford dipeptide 1 (Cbz-Ala-Ala-O-TAG1) in 87% yield.

(2) The above dipeptide 1 (Cbz-Ala-Ala-O-TAG1) was dissolved in ethyl acetate (4 mL), Pd(OH)₂/C (10 mol%) and Pd/C (10 mol%) were added, and the mixture was agitated under hydrogen gas (H₂) while heating to 50°C. After 6 hours, the mixture was filtered through filter paper with ethyl acetate (5 mL) and the filtrate was removed from the solvent using an evaporator. The operation described in (1) above was then performed again (except that the agitation was carried out for 19 hours). As a result, tripeptide 2 (Cbz-Ala-Ala-Ala-O-TAG1) was obtained in 84% yield.

### [Examples 2: Silicon-containing hydrophobic substituent TAG2(Si)]

### *Synthesis of TAG2(Si) reagent (TAG2(Si)-OH):

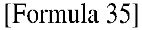

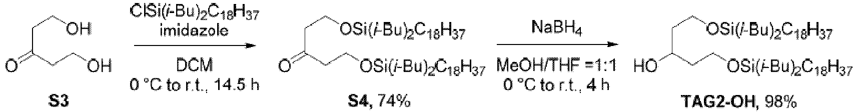

A diol compound of formula S3 (8.0 mmol, 1.0 equivalent) was added dropwise in dichloromethane (40 mL) at 0°C under a nitrogen atmosphere. Imidazole (24.0 mmol, 3.0 equivalents) was added, followed by the addition of chlorodibutyloctadecylsilane (16.0 mmol, 2.0 equivalents) with agitation at room temperature. After 14.5 hours, the mixture was partitioned via extraction with water and dichloromethane, and the organic phase was dried over sodium sulfate. The solvent was removed from the oil organic using an evaporator. The product was isolated by silica gel chromatography (hexane/ethyl acetate = 80/1) to give the compound of formula S4 in 74% yield (5.35 g).

The compound of formula S4 (5.9 mmol, 1.0 equivalent) was added dropwise to a methanol/tetrahydrofuran mixture at room temperature under a nitrogen atmosphere. The mixture was cooled to °C, and sodium borohydride (2.3 mmol, 2.0 equivalent) was added dropwise. The mixture was brought to room temperature, and agitated for 20 hours. Saturated sodium hydrogen chloride was added, and the mixture was partitioned via extraction with ethyl acetate. The organic phase was dried with sodium sulfate, and the solvent was removed using an evaporator. The product was isolated by silica gel chromatography (hexane/ethyl acetate = 70/1) to give the desired alcohol compound TAG2(Si)-OH in 98% yield (5.23 g).

### *Synthesis of an amino acid whose terminal carboxyl group was protected with TAG2(Si):

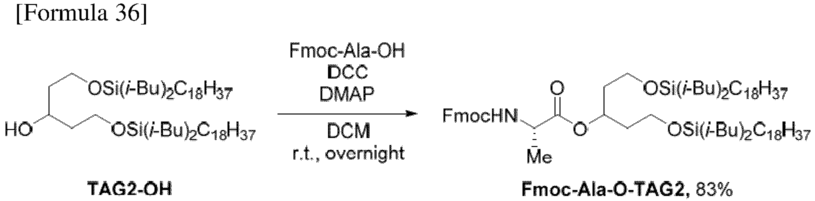

The alcohol of TAG2(Si) (0.30 mmol, 1.0 equivalent) was added dropwise at room temperature in dichloromethane (3.0 mL). Fmoc-Ala-OH (0.75 mmol, 2.5 equivalents) was added to the reaction mixture, followed by DMAP (0.36 mmol, 1.2 equivalents) and DCC (0.75 mmol, 2.5 equivalents). The mixture was then agitated at room temperature overnight, and filtered, and the solvent was removed using an evaporator. The product was isolated by silica gel chromatography (hexane/ethyl acetate = 60/1) to afford the desired Fmoc-Ala-O-TAG2(Si) in 83% yield (299.1 mg). The characterization data of the obtained Fmoc-Ala-O-TAG2(Si) are indicated below.

Rf = 0.23 (hexanes/EtOAc = 20:1).
[α]_{D}²⁴ = +6.31 (c 1.11, CHCl₃).
¹H NMR (400 MHz, CDCl₃) δ 7.77 (d, J = 7.5 Hz, 2H), 7.64 - 7.56 (m, 2H), 7.45 - 7.35 (m, 2H), 7.35 - 7.27 (m, 2H), 5.43 (d, J = 7.4 Hz, 1H), 5.21 - 5.08 (m, 1H), 4.44 - 4.31 (m, 3H), 4.28 - 4.17 (m, 1H), 3.67 - 3.52 (m, 4H), 1.87 - 1.75 (m, 8H), 1.43 (d, J = 7.1 Hz, 3H), 1.31 - 1.22 (m, 64H), 0.97 - 0.90 (m, 24H), 0.90 - 0.85 (m, 6H), 0.65 - 0.49 (m, 12H).
¹³C NMR (100 MHz, CDCl₃) δ 172.4, 155.4, 143.9, 143.8, 141.3, 127.7, 127.0, 125.1, 119.9, 71.2, 67.0, 58.8, 49.7, 47.2, 37.2, 33.8, 31.9, 29.71(8C), 29.66(2C), 29.4, 29.3, 26.4, 24.5, 24.2, 23.3, 22.7, 19.0, 14.8, 14.1.
IR (neat) 2951, 2921, 2852, 1728, 1504, 1450, 1380, 1364, 1330, 1206, 1086 cm⁻¹. HRMS (ESI) Calcd for C₇₅H₁₃₅NO₆S₁₂Na [M+Na]⁺: 1224.9726, Found: 1224.9705.

### *Peptide chain elongation reaction using the amino acid whose terminal carboxyl was protected with TAG2(Si) (1):

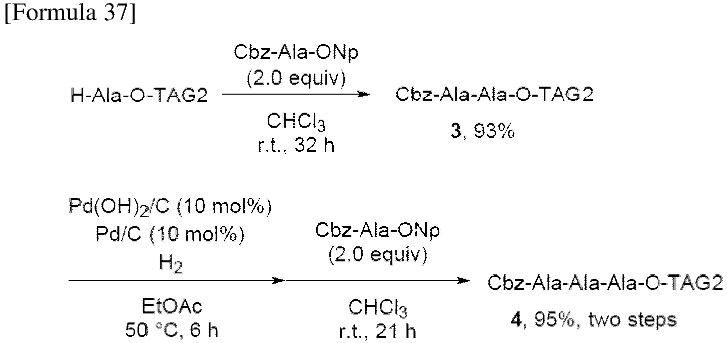

Synthesis of a tripeptide was examined using TAG2(Si). Specifically, the reaction was carried out as follows.

(1) A 6-mL vial was filled with a stirrer, H-Ala-O-TAG2 (0.45 mmol), chloroform (1 mL), and Cbz-Ala-ONp (2.0 equivalents; 0.9 mmol), sealed, and agitated at room temperature. After 32 hours, the mixture was partitioned via extraction with dichloromethane/water bicarbonate, the oil phase was dried with sodium sulfate, and the solvent was removed using an evaporator. The residue was isolated by column chromatography (hexane/ethyl acetate (EtOAc) = 7:1) to afford dipeptide 3 (Cbz-Ala-Ala-O-TAG2) in 93% yield.
(2) The above dipeptide 3 (Cbz-Ala-Ala-O-TAG2) was dissolved in ethyl acetate (4 mL), to which Pd(OH)₂/C (10 mol%) and Pd/C (10 mol%) were added, and the mixture was heated to 50°C under hydrogen gas (H₂) with agitation. After 6 hours, the mixture was filtered through filter paper with ethyl acetate (5 mL), and the solvent was removed from the filtrate using an evaporator. The operation described in (1) above was performed again (except that the agitation was carried out for 21 hours). As a result, tripeptide 4 (Cbz-Ala-Ala-Ala-O-TAG2) was obtained in 95% yield.

### *Peptide chain elongation reaction using the amino acid whose terminal carboxyl was protected with TAG2(Si) (2):

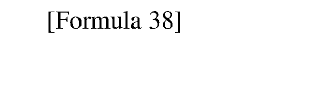

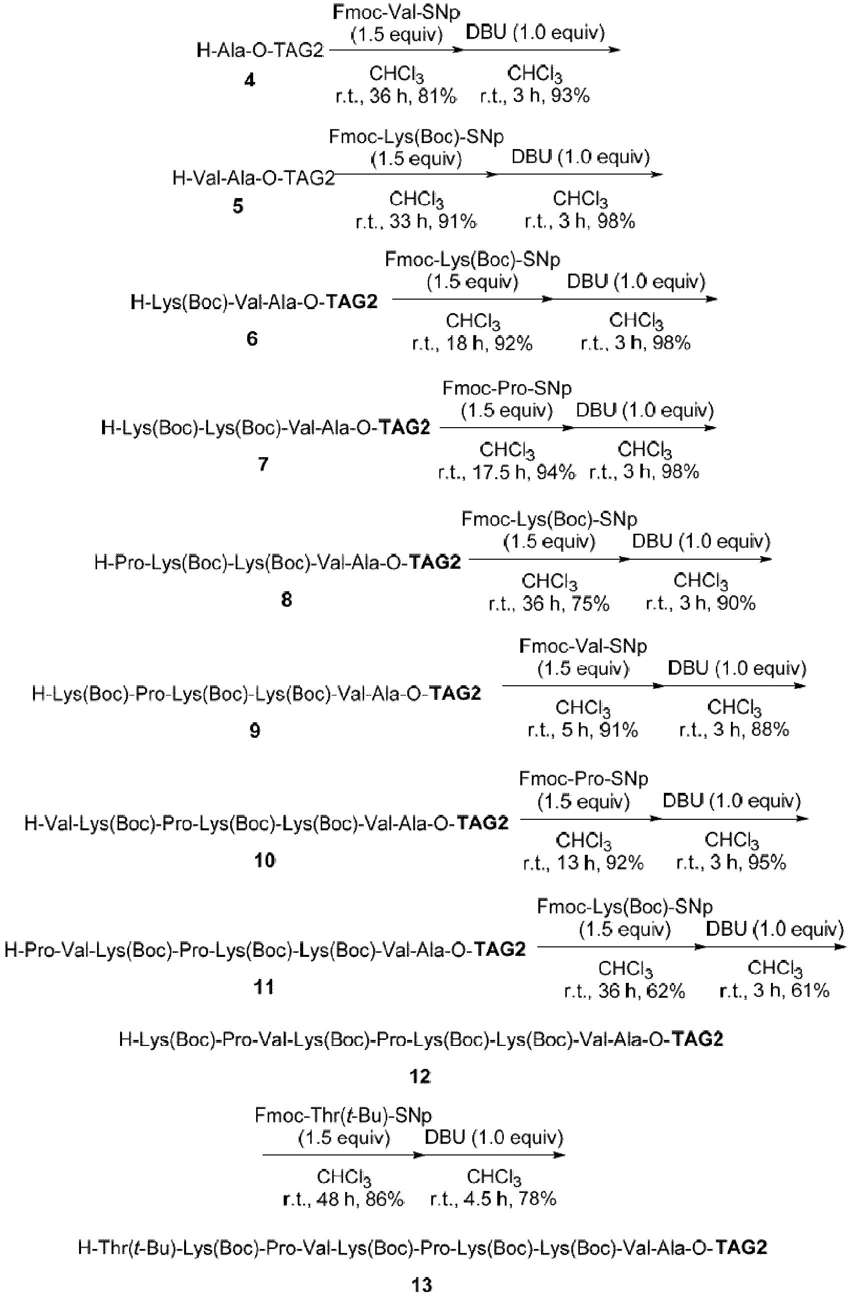

Synthesis of a tripeptide was examined using TAG2(Si). Specifically, the reaction was carried out as follows.

A 20-mL Nas flask was filled with a stirrer, H-Ala-O-TAG2 (2.4 mmol), chloroform (4.8 mL), and Fmoc-Val-SNp (3.6 mmol, SNp; p-nitrophenyl thioester), sealed, and agitated at room temperature. After 36 hours, the mixture was partitioned via extraction with dichloromethane/water bicarbonate, the organic phase was dried with sodium sulfate, and the solvent was removed using an evaporator. The residue was isolated by column chromatography (hexane/ethyl acetate (EtOAc) = 10: 1) to give the dipeptide (Fmoc-Val-Ala-O-TAG2) in 81% yield. The resulting product was dissolved in chloroform (21 mL), mixed with DBU (1,8-diazabicyclo[5.4.0]undese-7-ene; 1 equivalent), and agitated at room temperature. After 3 hours, the residue was isolated by column chromatography (hexane/ethyl acetate (EtOAc) = 10:1) to give dipeptide 2 (H-Val-Ala-O-TAG2) in 93% yield.

The above operations were repeated with changing the added protective amino acid (Fmoc-AA-SNp: AA is an amino acid residue) as shown in the above reaction formula to give decapeptide 13 (H-Thr(t-Bu)-Lys(Boc)-Pro-Val-Lys(Boc)-Pro-Lys(Boc)-Lys(Boc)-Val-Ala-O-TAG2) as the final product in 78% yield.

### [Examples 3: Silicon-containing hydrophobic substituent TAG3(Si)]

### *Synthesis of TAG3(Si) reagent (TAG3(Si)-OH):

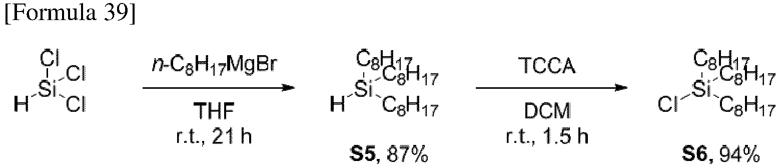

A piece of magnesium (51.0 mmol, 5.1 equivalents) was added to tetrahydrofuran (60mL) at room temperature under a nitrogen atmosphere, and bromooctane (34.0 mmol, 3.4 equivalents) was added to the mixture dropwise. After 10 minutes, the mixture was agitated for 2 hours with reflux. The mixture was transferred to an ice bath, and trichlorosilane (10.0 mmol, 1.0 equivalent) was added to the mixture dropwise. The mixture was returned to room temperature, and agitated for 21 hours. The mixture was mixed with saturated ammonium chloride solution, and partitioned via extraction with hexane. The organic phase was dried over sodium sulfate and evaporated. The product was isolated by silica gel chromatography (hexane) to afford the compound of formula S5 in 87% yield (3.21 g).

The compound of formula S5 (10.0 mmol, 1.0 equivalent) was added dropwise in dichloromethane (100 mL) under a nitrogen atmosphere, and trichloroisocyanuric acid (3.4 mmol, 0.34 equivalent) was added to the mixture dropwise. The mixture was agitated at room temperature for 1.5 hours, and the solvent was removed to afford the compound of formula S6 in 94% yield (3.79 g).

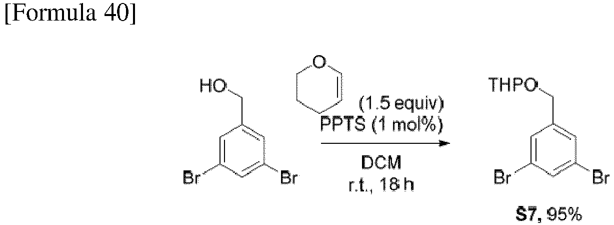

3,5-Dibromobenzyl alcohol (10.0 mmol, 1.0 equivalent) and pyridinium p-toluenesulfonate (1.0 mmol, 0.1 equivalent) were added in dichloromethane (50 mL) at room temperature. The mixture was combined with 3,4-dihydro-2H-pyran (15.0 mmol, 1.5 equivalents), and agitated at room temperature. After 18 hours, the mixture was partitioned via extraction with sodium bicarbonate water and dichloromethane, the organic phase was dried with sodium sulfate, and the solvent was evaporated off with an evaporator. The product was isolated by silica gel chromatography (hexane/ethyl acetate = 20/1) to afford the compound of formula S7 in 95% yield (3.32 g).

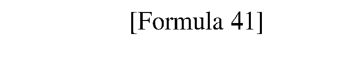

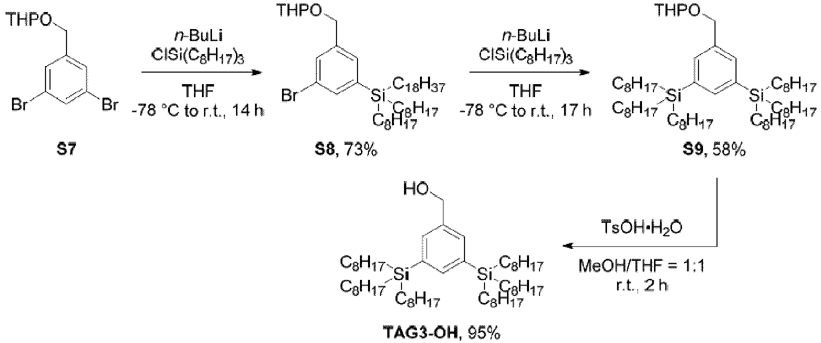

The compound of formula S7 (4.0 mmol, 1.0 equivalent) was added in tetrahydrofuran (24 mL) at -78°C under a nitrogen atmosphere, followed by the addition n-butyl lithium (1.57 M in hexane, 2.7 mL, 4.2 mmol, 1.05 equivalent). After 1 hour at - 78°C, a chlorotrioctylsilane compound of formula S6 (2.26 g, 5.6 mmol, 1.4 equivalents) was added, and the mixture was brought to room temperature and agitated for 14 hours. The product was isolated by silica gel chromatography (hexane/ethyl acetate = 75/1) to afford the compound of formula S8 in 73% yield (1.85 g).

The compound of formula S8 was added in tetrahydrofuran (24 mL) at -78°C under a nitrogen atmosphere. n-Butyl lithium (1.57 M in hexane, 1.9 mL, 3.1 mmol, 1.1 equivalents) was added. The mixture was agitated for 1.5 hours, and mixed with the chlorotrioctylsilane compound of formula S6 (1.51 g, 3.7 mmol, 1.35 equivalents). The mixture was returned to room temperature, and agitated for 17 hours. The solvent was removed, and the product was isolated by silica gel chromatography (hexane/ethyl acetate = 85/1) to give the compound of formula S9 in 58% yield (1.54 g).

The compound of formula S9 was added to methanol /tetrahydrofuran (8mL/8mL) at room temperature, and mixed with p-toluene sulfonic acid (31.8mg, 0.167mmol, 0.1 equivalents). The mixture was agitated at room temperature for 2 hours, and the solvent was removed. The product was isolated by silica gel chromatography (hexane /acetic acid ethyl =85/1) to afford the target alcohol compound TAG3(Si)-OH in 95% yield (1.33g).

*Synthesis of an amino acid whose terminal carboxyl protection 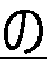 synthesis :

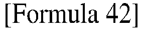

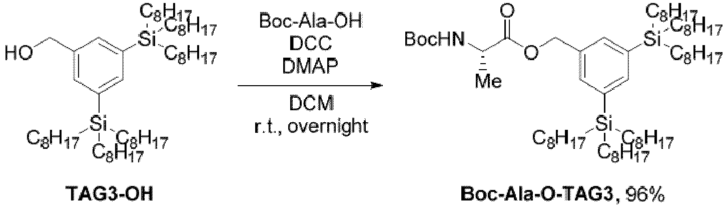

The alcohol of TAG3(Si) (0.08mmol, 1.0 equivalents) was added to dichloromethane (1.0mL) dropwise at room temperature. The reaction solution was mixed with Boc-Ala-OH (0.16mmol, 2.0 equivalents), and then with DMAP (0.096mmol, 1.2 equivalents) and DCC (0.16mmol, 2.0 equivalents). The mixture was agitated overnight at room temperature, and then filtered with a filter paper, and the solvent was removed using an evaporator. The product was isolated by silica gel chromatography (hexane /acetic acid ethyl =40/1) to afford Boc-Ala-O-TAG3(Si) in 96% yield (77.8mg).

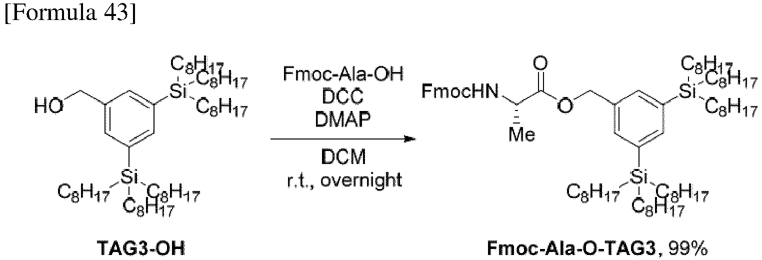

The alcohol of TAG3(Si) (1.58mmol, 1.0 equivalents) was added dropwise to dichloromethane (16.0mL) at room temperature. The reaction solution was mixed with Fmoc-Ala-OH (3.16mmol, 2.0 equivalents), and then with DMAP (1.90mmol, 1.2 equivalents) and DCC (3.16mmol, 2.0 equivalents). The mixture was agitated overnight at room temperature, and filtered with a filter paper. The solvent was removed using an evaporator. The product was isolated by silica gel chromatography (hexane /acetic acid ethyl =40/1) to afford Fmoc-Ala-O-TAG3(Si) in 99% yield (1.78g). The characterization data of the obtained Fmoc-Ala-O-TAG3(Si) are indicated below.

Rf = 0.20 (hexanes/EtOAc = 20:1).
[α]_{D}²⁴ = +2.80 (c 1.07, CHCl₃).
¹H NMR (400 MHz, CDCl₃) δ 7.77 (d, J = 7.5 Hz, 2H), 7.66 - 7.55 (m, 3H), 7.45 - 7.36 (m,
4H), 7.36 - 7.28 (m, 2H), 5.43 (d, J = 7.7 Hz, 1H), 5.28 (d, J = 12.2 Hz, 1H), 5.13 (d, J = 12.2 Hz, 1H), 4.54 - 4.37 (m, 3H), 4.24 (t, J = 7.2 Hz, 1H), 1.46 (d, J = 7.1 Hz, 3H), 1.36 - 1.23 (m, 72H), 0.93 - 0.84 (m, 18H), 0.84 - 0.73 (m, 12H).
¹³C NMR (100 MHz, CDCl₃) δ 172.9, 155.6, 143.9, 143.8, 141.3, 140.3, 137.5, 134.1, 133.3, 127.7, 127.0, 125.1, 120.0, 67.9, 67.0, 49.7, 47.1, 33.8, 31.9, 29.30, 29.27, 23.8, 22.7, 18.8, 14.1, 12.4.
IR (neat) 2955, 2920, 2852, 1728, 1505, 1451, 1336, 1201, 1173, 1106, 1074, 1054 cm⁻¹. HRMS (ESI) Calcd for C₇₃H₁₂₃NO₄S₁₂Na [M+Na]⁺: 1156.8888, Found: 1156.8890.

### *Peptide chain elongation reaction using the amino acid whose terminal carboxyl was protected with TAG3(Si):

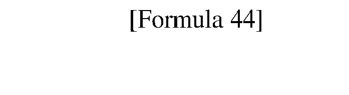

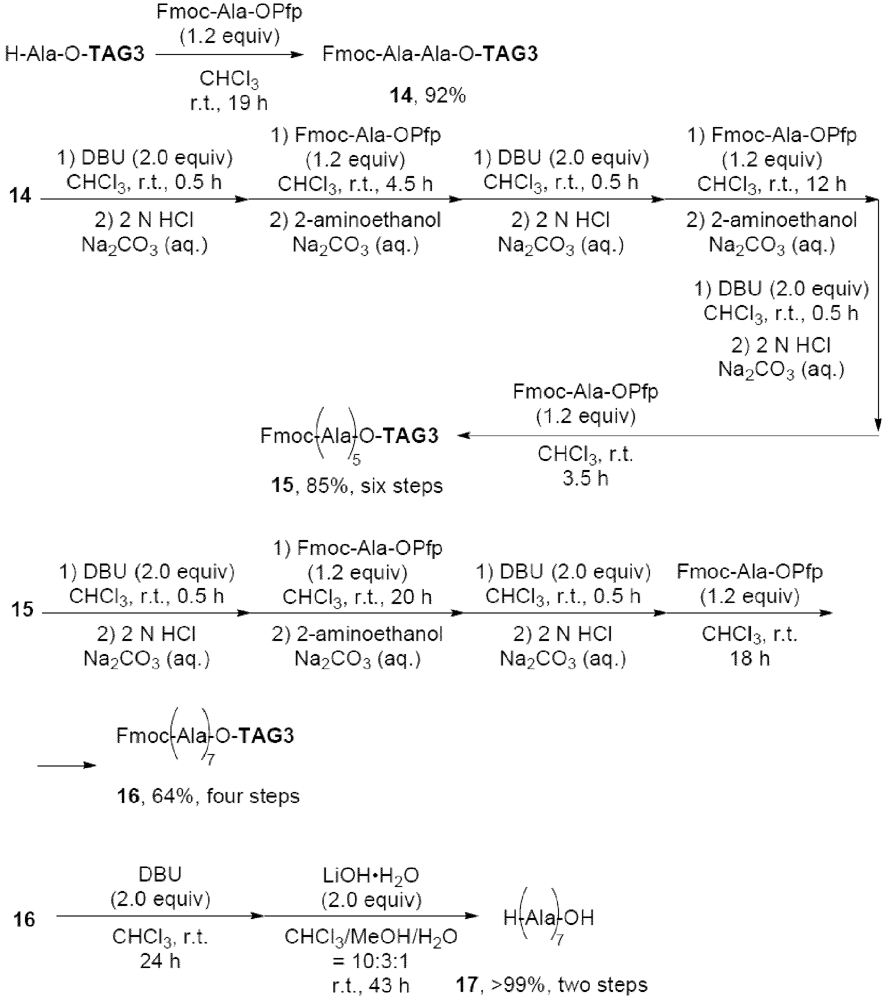

A sequential elongation reaction was examined using an amino acid whose terminal carboxyl group was protected with TAG3(Si). Specifically, the reaction was carried out as follows.

(1) 6mL vial was filled with a stirrer, H-Ala-O-TAG3 (1.1mmol), chloroform (2.2mL), and Fmoc-Ala-OPfp (1.32mmol, Pfp; pentafluorophenyl), sealed, and agitated at room temperature. After 19 hours, 2-amino ethanol (110µL) was added to the mixture to inactivate the extra Fmoc-Ala-OPfp. The mixture was partitioned via extraction with dichloromethane/water, the organic phase was dried with sodium sulfate, and the solvent was removed using an evaporator. The residue was isolated by column chromatography (hexane/ethyl acetate (EtOAc) = 5:1) to give dipeptide 14 (Fmoc-Ala-Ala-O-TAG3) in 92 yield % (1.22 g).
(2) The dipeptide 14 (Fmoc-Ala-Ala-O-TAG3) was dissolved in chloroform (0.1M), mixed with DBU (1,8-diazabicyclo[5.4.0]undec-7-ene; 2 equivalents), and agitated at room temperature. After 30 minutes, hydrochloric acid (2N, 1 equivalent) was added to the mixture, was confirmed to have a pH of 6 to 7 using a pH test paper. The mixture was partitioned via extraction with aqueous solution of sodium carbonate and dichloromethane, the organic phase was dried with sodium sulfate, and the solvent was removed using an evaporator.
(3) The operations of (1) and (2) were repeated to thereby afford pentapeptide 15(Fmoc-Ala-Ala-Ala-Ala-Ala-O-TAG3) in 85% yield. The operations of (1) and (2) were further repeated twice to thereby afford heptapeptide 16 (Fmoc-Ala-Ala-Ala-Ala-Ala-Ala-O-TAG3) in 64% yield.
(4) The heptapeptide 16 (Fmoc-Ala-Ala-Ala-Ala-Ala-Ala-O-TAG3) (0.024mmol) was dissolved in chloroform (0.5mL), mixed with DBU (2 equivalents), and agitated at room temperature. After 2 hours, 1mL of chloroform was added to the mixture, which was then agitated further at room temperature for 22 hours. The solvent was removed using an evaporator, and the residue was mixed with chloroform (0.5mL), methanol (0.15mL), and water (50µL), and then with lithium hydroxide monohydrate (2 equivalents), and agitated at room temperature. After 43 hours, the solvent was removed, hydrochloric acid (2N, 2.07 equivalents) was added to the residue, and the solid was filtered with water and chloroform. The obtained solid was dissolved in trifluoroacetic acid, the unwanted material was filtered off, and the solvent was removed, to thereby afford septapeptide 17 (Fmoc-Ala-Ala-Ala-Ala -Ala-Ala-Ala-Ala-O-TAG3) in over 99% yield.

### *Synthesis of an amino acid whose terminal amino was protected with TAG3(Si):

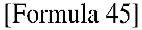

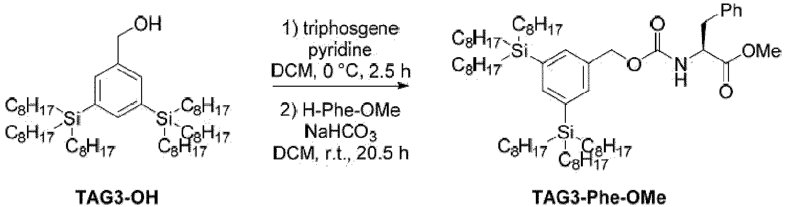

Triphosgene (0.1 mmol) was added dropwise in dichloromethane (2 mL) at 0°C under a nitrogen atmosphere. A dichloromethane solution (2mL) of the alcohol of TAG3(Si) (0.2mmol) and pyridine (0.2 mmol) was added slowly. After 2.5 hours, the mixture was returned to room temperature, and dropped into a dichloromethane solution (2 mL) of sodium bicarbonate and phenylalanine methyl ester, which had been cooled to 0°C. After 30 minutes, the mixture was returned to room temperature, and agitated for 20.5 hours. The reaction solution was filtered, the solvent was removed using an evaporator, and the residue was isolated by column chromatography (hexane /acetic acid ethyl (EtOAc)=80:1 --> 50:1 --> 20:1) to afford TAG3-Phe-OMe in 76% yield (160mg). The characterization data of the obtained TAG3-Phe-OMe are indicated below.

Rf = 0.21 (hexanes/EtOAc = 20:1).
[α]_{D}²⁵ = +25.74 (c 1.01, CHCl₃).
¹H NMR (400 MHz, CDCl₃) δ 7.56 (s, 1H), 7.40 (s, 2H), 7.31 - 7.20 (m, 3H), 7.16 - 7.03 (m, 2H), 5.22 (d, J = 8.2 Hz, 1H), 5.15 - 5.03 (m, 2H), 4.76 - 4.64 (m, 1H), 3.72 (s, 3H), 3.22 - 3.02 (m, 2H), 1.38 - 1.19 (m, 72H), 0.95 - 0.84 (m, 18H), 0.83 - 0.71 (m, 12H). ¹³C NMR (100 MHz, CDCl₃) δ 171.9, 155.7, 140.2, 137.3, 135.7, 134.5, 133.9, 129.2, 128.6, 127.1, 67.9, 54.8, 52.2, 38.3, 33.8, 31.9, 29.32, 29.26, 23.9, 22.7, 14.1, 12.5. IR (neat) 2955, 2920, 2852, 1726, 1499, 1457, 1377, 1346, 1205, 1177, 1144 cm⁻¹. HRMS (ESI) Calcd for C₆₆H₁₁₉NO₄S₁₂Na [M+Na]⁺: 1068.8575, Found: 1068.8540.

### *Peptide chain elongation reaction using the amino acid whose terminal amino was protected with TAG3(Si):

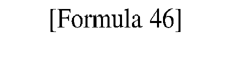

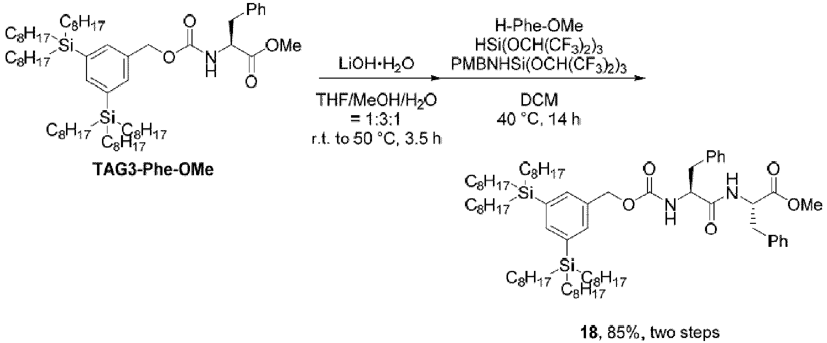

A sequential elongation reaction was examined using an amino acid whose terminal amino group was protected with TAG3(Si). Specifically, the reaction was carried out as follows.

The TAG3-Phe-OMe above (0.1mmol) and lithium hydroxide monohydrate (1 equivalents) was added to THF/MeOH/H₂O (0.1mL/0.3mL/0.1mL) and agitated at room temperature for 1.5 hours, and at 50 °C for additional 2 hours. The resulting solution was diluted with chloroform (2mL), mixed with hydrochloric acid (2N, 1 equivalent), and partitioned via extraction with water/chloroform. the organic phase was dried with sodium sulfate, and the solvent was removed using an evaporator. In a glove box, the resulting residue was combined with a dichloromethane (0.1mL), HSi[OCH(CF₃)₂]₃ (1.5 equivalents), H-Phe-OMe (1.5 equivalents), and PMBNHSi[OCH(CF₃)₂]₃ (1.0M indichloromethane, 0.03 equivalents) and sealed in a vial, which was taken out of the glove box. This reaction solution was agitated at 40 °C for 14 hours. The reaction product was isolated by silica gel chromatography (hexane /EtOAc=7:1) to afford dipeptide 18 (TAG3-Phe-Phe-OMe) in 85% yield. The characterization data of the resulting dipeptide 18 are indicated below.

Rf = 0.34 (hexanes/EtOAc = 5:1).
[α]_{D}²² = +20.20 (c 0.99, CHCl₃).
¹H NMR (400 MHz, CDCl₃) δ 7.57 (s, 1H), 7.41 (s, 2H), 7.33 - 7.10 (m, 8H), 7.05 - 6.92 (m, 2H), 6.20 (d, J = 7.3 Hz, 1H), 5.25 (d, J = 8.0 Hz, 1H), 5.18 - 4.94 (m, 2H), 4.86 - 4.74 (m, 1H), 4.52 - 4.35 (m, 1H), 3.68 (s, 3H), 3.22 - 2.92 (m, 4H), 1.42 - 1.16 (m, 72H), 0.97 - 0.84 (m, 18H), 0.84 - 0.68 (m, 12H).
¹³C NMR (100 MHz, CDCl₃) δ 171.2, 170.3, 155.9, 140.2, 137.4, 136.2, 135.5, 134.4, 133.8, 129.3, 129.2, 128.7, 128.5, 127.1, 127.0, 68.0, 56.1, 53.3, 52.3, 38.3, 37.9, 33.8, 31.9, 29.32, 29.26, 23.8, 22.7, 14.1, 12.5.
IR (neat) 2955, 2920, 2852, 1746, 1711, 1664, 1498, 1456, 1377, 1213, 1177, 1144, 1109, 1078, 1031 cm⁻¹.
HRMS (ESI) Calcd for C₇₅H₁₂₈N₂O₅S₁₂Na [M+Na]⁺: 1215.9259, Found: 1215.9275.

### [Reference Example: Evaluation of protecting groups for the terminal carboxyl of amino acids or peptides]

As protecting groups for the terminal carboxyl groups of amino acid/peptide compounds, the silicon-containing hydrophobic substituents TAG1(Si), TAG2(Si), and TAG3(Si) synthesized in Examples 1 to 3, the conventionally known protecting group tBu group, and the previously mentioned TAGa group (alkoxybenzyl ester group) represented by the following formula, which is described in Non-Patent Literature 6 (Org. Process Res. Dev. 2019, Vol. 23, p. 2576-2581), were used to protect the terminal carboxyl of an amino acid (L-Ala), and their effects on polarity reduction were evaluated.

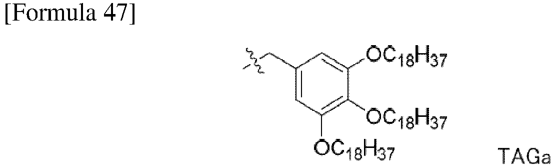

According to a usual method, the terminal carboxyl of L-Ala was protected with PG (where PG was the above-mentioned TAG1(Si), TAG2(Si), TAG3(Si), tBu, or TAGa) to prepare H-Ala-O-PG, and the resulting compounds were subjected to TLC analysis with a polarity of hexane/ethyl acetate = 2/1. The results are shown in the graph in Figure 1. In the case of the conventional protecting group tBu, which is commonly used in dipeptide synthesis and other applications, the Rf value was 0.07. In the case of the protecting group TAGa, which is described in Non-Patent Literature 6, the Rf value was 0.16. On the other hand, in the case of the TAG 1 (Si) group of Example 1, the TAG2(Si) group of Example 2, and the TAG3(Si) group of Example 3, which are the protective groups of the present invention, the Rf values were 0.44, 0.64, and 0.61, respectively, indicating that a significant decrease in polarity was confirmed. These results indicate that the silicon-containing hydrophobic substituents TAG(Si) are superior in decreasing the polarity of amino acids or peptides. It should also be noted that when the protecting group TAGa described in Non-Patent Literature 6 was used, an insoluble solid was observed in the reaction process, suggesting that the terminal carboxyl may be deprotected in various reactions.

## Claims

1. A method for producing a polypeptide compound, comprising the step of causing an amide forming reaction between the carboxyl group on the right side of an amino acid or peptide compound represented by formula (R1) and the amino group on the left side of represented by formula (R2) amino acid ester or peptide ester compound to produce a peptide compound represented by formula (P1). 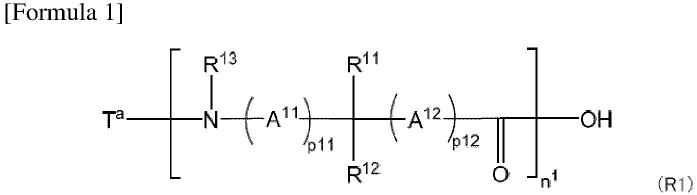 In formula (R1),
T^{a} represents a hydrogen atom or monovalent substituent,
R¹¹ and R¹² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
R¹³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R¹¹ and R¹³ may be bound to each other to form, together with the carbon atom to which R ¹¹ binds and the nitrogen atom to which R¹³ binds, a heterocyclic group that may have one or more substituents,
A¹¹ and A¹² each represent, independently of each other, a divalent aliphatic hydrocarbon group that may have one or more substituents having 1 to 3 carbon atoms,
p11 and p12 each represent, independently of each other, 0 or 1, and
n¹ represents an integer of equal to or greater than 1 corresponding to the number of the structure units parenthesized with [ ], provided that when n¹ is equal to or greater than 2, then the two or more structure units in [ ] may be either identical to each other or different from each other. 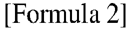 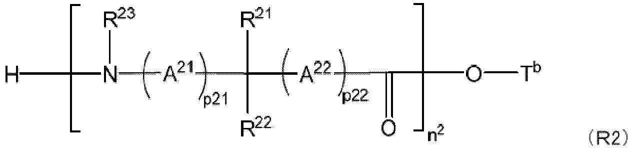
In formula (R2),
T^{b} represents a hydrogen atom or monovalent substituent,
R²¹and R²² each represent, independently of each other, a hydrogen atom, halogen atom, hydroxyl group, carboxyl group, nitro group, cyano group, or thiol group, or an amino group, monovalent aliphatic hydrocarbon group, monovalent aromatic hydrocarbon group, or monovalent heterocyclic group that may have one or more substituents,
R²³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R²¹ and R²³ may be bound to each other to form, together with the carbon atom to which R²¹ binds and the nitrogen atom to which R²³ binds, a heterocyclic group that may have one or more substituents,
A²¹ and A²² each represent, independently of each other, a divalent aliphatic hydrocarbon group that may have one or more substituents having 1 to 3 carbon atoms,
p21 and p22 each represent, independently of each other, 0 or 1, and
n² represents an integer of equal to or greater than 1 corresponding to the number of the structure units parenthesized with [ ], provided that when n² is equal to or greater than 2, then the two or more structure units in [ ] may be either identical to each other or different from each other, provided that
(i) T^{a} is a group represented by -O-C(=O)-TAG(Si) and/or
(ii) T^{b} is a group represented by -TAG(Si),
where -TAG(Si) has the following structure. 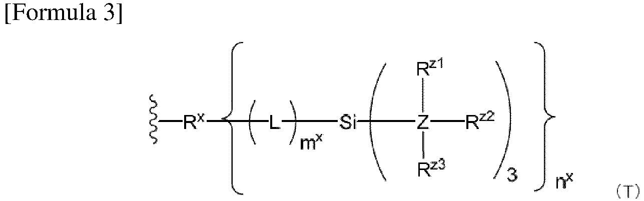
In formula (T),
R^{x} represents a divalent, trivalent, or tetravalent aliphatic hydrocarbon group, aromatic hydrocarbon group, or heterocyclic group that may have one or more substituents,
L represents a single bond or a divalent linking group,
m^{x} represents an integer of 0 or 1 provided that when m^{x} is 0, L is a single bond,
Z represent, independently of each other, a carbon atom (C) or a silicon atom (Si), R^{z1}, R^{z2}, and R^{z3} represent, independently of each other, a monovalent aliphatic hydrocarbon group, aromatic hydrocarbon group, silicon-containing hydrocarbon group, or heterocyclic group that may have one or more substituents,
provided that the three parenthesized substituents in -Z(R^{z1})(R^{z2})(R^{z3}) may either be the same as each other or different from each other,
n^{x} represents an integer of 1 to 3 corresponding to the number of the substituents parenthesized with { }, provided that when m^{x} is 2 or 3, then the two or three substituents parenthesized with { } in formula (T) may either be the same as each other or different from each other. 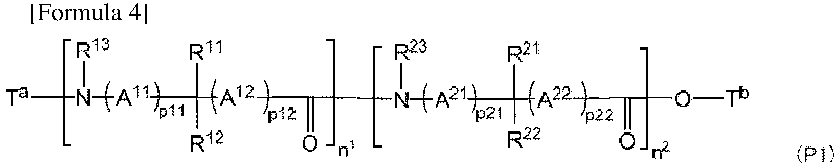
In formula (P1),
T^{a}, R¹¹, R¹², R¹³, A¹¹, A¹², p11, p12, and n¹ each represent the same definitions as those of the same symbols in general formula (R1) above, and
R²¹, R²², R²³, A²¹, A²², p21, p22, n², and T^{b} each represent the same definitions as those of the same symbols in general formula (R2) above.

2. The method according to claim 1, wherein -TAG(Si) has a structure represented by formula (T1), (T2), or (T3) below. 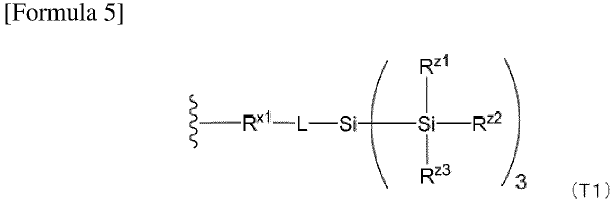 In formula (T1),
R^{x1} represents a divalent aliphatic hydrocarbon group, aromatic hydrocarbon group, or heterocyclic group that may have one or more substituents, and
L, R^{z1}, R^{z2}, and R^{z3} represent, independently of each other, the same definitions as in formula (T). 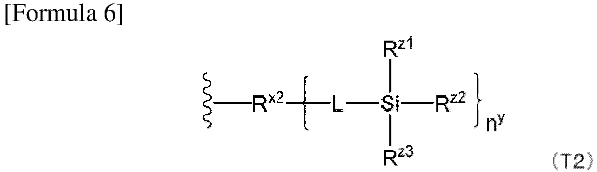
In formula (T2),
R^{x2} represents a trivalent or tetravalent aliphatic hydrocarbon group, aromatic hydrocarbon group, or heterocyclic group that may have one or more substituents,
L, R^{z1}, R^{z2}, and R^{z3} represent, independently of each other, the same definitions as in formula (T), and
n^{y} represents an integer of 2 or 3 corresponding to the number of the substituents parenthesized with { }, provided that the two or three substituents parenthesized with { } in formula (T2) may either be the same as each other or different from each other . 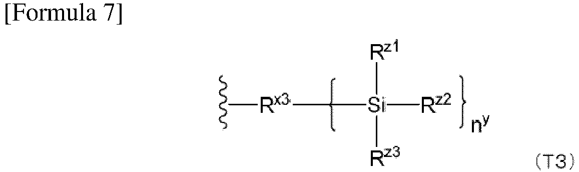
In formula (T3),
R^{x3} represents a trivalent or tetravalent aliphatic hydrocarbon group, aromatic hydrocarbon group, or heterocyclic group that may have one or more substituents,
R^{z1}, R^{z2}, and R^{z3} represent, independently of each other, the same definitions as in formula (T), and
n^{y} represents an integer of 2 or 3 corresponding to the number of the substituents parenthesized with { }, provided that the two or three substituents parenthesized with { } in formula (T3) may either be the same as each other or different from each other.

3. The method according to claim 1 or 2, wherein the reaction is carried out in an organic solvent.

4. The method according to any one of claims 1 to 3, wherein the reaction is carried out in the presence of a Lewis acid catalyst.

5. The method according to claim 4, wherein the Lewis acid catalyst is a metal compound containing one or more metals selected from the group consisting of aluminum, titanium, zirconium, hafnium, tantalum, and niobium.

6. The method according to any one of claims 1 to 5, wherein the reaction is carried out in the presence of a phosphorus compound.

7. The method according to claim 6, wherein the phosphorus compound is a phosphine compound or a phosphate compound.

8. The method according to any one of claims 1 to 7, wherein the reaction is carried out in the presence of a silane compound.

9. The method according to any one of claims 1 to 8, wherein the T^{a} group in formula (R1) is -O-C(=O)-TAG(Si), and the method further comprises the step of, after the synthesis of the peptide compound of formula (P1), deprotecting the terminal amino group of the peptide compound by removing the -O-C(=O)-TAG(Si) group from the peptide compound.

10. The method according to any one of claims 1 to 9, wherein the T^{b} group in formula (R1) is -TAG(Si), and the method further comprises the step of, after the synthesis of the peptide compound of formula (P1), deprotecting the terminal carboxyl group of the peptide compound by removing the -TAG(Si) group from the peptide compound.

11. The method according to any one of claims 1 to 10, wherein the reaction is carried out as a batch reaction or a flow reaction.

12. An amino acid or peptide compound represented by formula (R1') below. 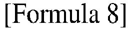 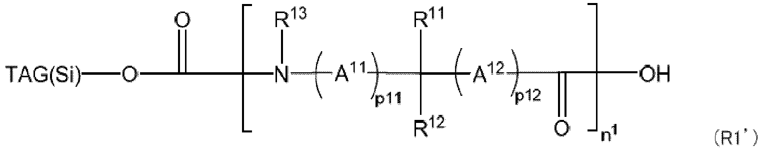 In formula (R1'), R¹¹, R¹², R¹³, A¹¹, A¹², p11, p12, n¹, and TAG(Si) each represent the same definitions as those of the same symbols in claim 1.

13. An amino acid ester or peptide ester compound represented by formula (R2') below. 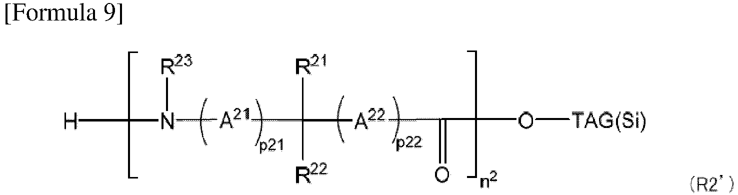 In formula (R2'), R²¹, R²², R²³, A²¹, A²², p21, p22, n², and TAG(Si) each represent the same definitions as those of the same symbols in claim 1.

14. A peptide compound represented by formula (P1') below. 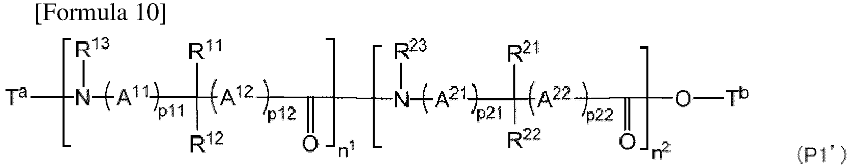 In formula (P1'),
R¹¹, R¹², R¹³, R²¹, R²², R²³, A¹¹, A¹², A²¹, A²², p21, p22, p11, p12, n¹, n², T^{a}, and T^{b} each represent the same definitions as those of the same symbols in claim 1,
provided that
(i) T^{a} is a group represented by -O-C(=O)-TAG(Si), and/or,
(ii) T^{b} is a group represented by -TAG(Si),
where -TAG(Si) represents the same definition as in claim 1.

15. A compound represented by formula (C0) below.
[Formula 11]
TAG(Si)-OH (C0)
In formula (C0), -TAG(Si) represents the same definition as the same symbol in claim 1.

16. A compound represented by formula (C1) below. 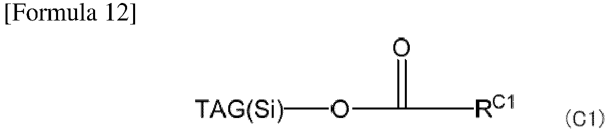 In formula (C1),
-TAG(Si) represents the same definition as the same symbol in claim 1, and
R^{C1} represents a monovalent group.

17. A compound represented by formula (C2) below. 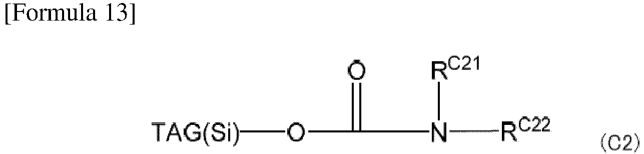 In formula (C2),
-TAG(Si) represents the same definition as the same symbol in claim 1, and
R^{C21} and R^{C22} represent, independently of each other, a monovalent group.
